# EUROPEAN PATENT APPLICATION

(11) **EP 2 149 558 A1**
(43) Date of publication of application: **03.02.2010**
(21) Application number: 08075651.3
(22) Date of filing: 21.07.2008
(51) Int. Cl.: C07D 215/38, C07D 401/04, A61K 31/4706, A61K 31/4709, A61P 29/00

(54) **5-[(3,3,3-Trifluoro-2-hydroxy-1-arylpropyl)amino-1-arylquinolin-2-ones, a process for their production and their use as anti-inflammatory agents**

(71) Applicant: Bayer Schering Pharma Aktiengesellschaft, 13353 Berlin (DE); AstraZeneca AB, 151 85 Södertälje (SE)
(72) Inventor: Merkus Berger, Dr., 13347 Berlin (DE); Hartmut Rehwinkel, Dr., 10961 Berlin (DE); Heike Schäcke, Dr., 10115 Berlin (DE); Ekkehard May, 13465 Berlin (DE)

(57) **Abstract**

The present invention relates to compounds of formula I, 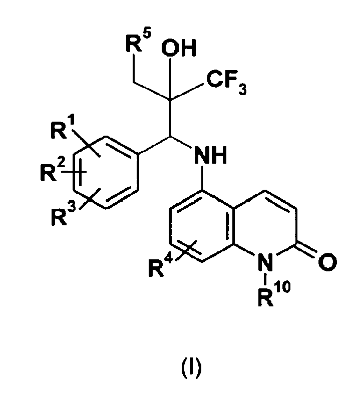 processes and intermediates for their production and their use as antiinflammatory agents.

## Description

The present invention relates to compounds of formula I, a process for their production and their use as anti-inflammatory agents.

The most common anti-inflammatory agents are still the glucocorticoids (GCs) which are small molecules having a steroidal structure that interact with the glucocorticoid receptor (GR), whether endogenous, like cortisol, or synthetic, like dexamethasone and others. However, the application of highly potent GCs, especially over long treatment periods, led to the occurrence of undesired effects. A number of these effects, are severe and sometimes irreversible (Schäcke et al., 2002 Pharmacol. & Therapeutics (2002) 96(1):23-43., Miner et al., 2005 Expert Opin. Investig. Drugs (2005) 14(12):1527-1545.)The GCs potently inhibit pro-inflammatory cytokines and chemokines at the site of administration, whereas they elicit only limited systemic effects (O'Connell, 2003 Clin. Ther. (2003) 25(Suppl. C):C42-60; Welker et al. Int. Arch. Allergy Immunol. (1996) 109(2):110-115, 1996, Günther et al., 1998. Skin Pharmacol. Appl. Skin Physiol. (1998) 11(1):35-42). Although locally active GCs appeared to be the ideal anti-inflammatory drugs, their application is limited due to local side effects and to insufficient efficacy in severe disease states.

Therefore, there is a great medical need for new compounds that have anti-inflammatory / immunomodulatory activity similar to the marketed GCs, and are less likely to produce undesired effects.

From the prior art of DE 100 38 639 and WO 02/10143, anti-inflammatory agents of the following general formula 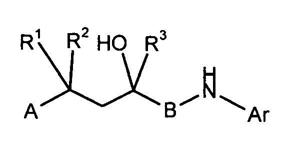 are known, wherein the Ar radical comprises phthalides, thiophthalides, benzoxazinones or phthalazinones. In the experiment, these compounds show dissociations of action between anti-inflammatory and undesirable metabolic actions and are superior to the previously described nonsteroidal glucocorticoids or exhibit at least just as good an action.
Compounds structurally related to those described in this patent application are disclosed in WO 2005/035518 and EP 1878717 A1.
The compounds of the present invention are different with regard to the substituents of the chinolinone.
It was therefore the object of this invention to make compounds available whose potency of anti inflammatory effects is at least as good as the potency of the state of the art compounds.
This object has been achieved by the compounds according to the claims.

This invention therefore relates to compounds of general formula I 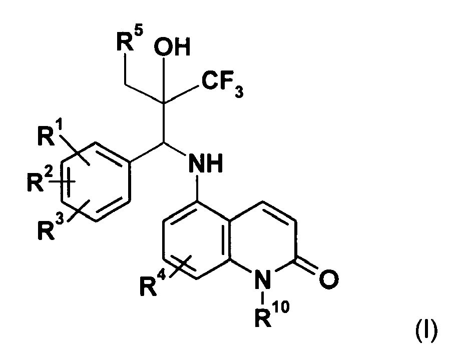 in which
- R¹and R²: independently of one another, mean a hydrogen atom, a hydroxy group, a halogen atom, an optionally substituted (C₁-C₁₀)-alkyl group, an optionally substituted (C₁-C₁₀)-alkoxy group, a (C₁-C₁₀)- alkylthio group, a (C₁-C₅)-perfluoroalkyl group, a cyano group, a nitro group,
or R¹ and R²
together mean a group that is selected from the groups -O-(CH₂)ₚ- O-, -O-(CH₂)ₚ-CH₂-, -O-CH=CH-, -(CH₂)ₚ₊₂-, -NH-(CH₂)ₚ₊₁, -N(C₁- C₃-alkyl)-(CH₂)ₚ₊₁, and -NH-N=CH-,
whereby p = 1 or 2, and the terminal oxygen atoms and/or carbon atoms and/or nitrogen atoms are linked to directly adjacent ring-carbon atoms,
or NR⁶R⁷,
whereby R⁶ and R⁷, independently of one another, mean hydrogen, C₁-C₅-alkyl or (CO)-(C₁-C₅)-alkyl,
- R³: means a hydrogen atom, a hydroxy group, a halogen atom, a cyano group, an optionally substituted (C₁-C₁₀)-alkyl group, a (C₁- C₁₀)-alkoxy group, a (C₁-C₁₀)-alkylthio group, or a (C₁-C₅)- perfluoroalkyl group,
- R⁴: means a hydrogen, halogen, hydroxy, (C₁-C₅)-alkyl, (C₁-C₅)alkoxy, (C₁-C₅)-alkylthio, (C₁-C₅)-perfluoroalkyl, cyano, nitro, NR⁷R⁸, COOR⁹, (CO)NR⁷R⁸ or a (C₁-C₅-alkylene)-O-(CO)-(C₁-C₅)alkyl group
- R⁵: means a group selected from
-(C₁-C₁₀)alkyl, which may be optionally partially or completely halogenated,
-(C₁-C₁₀)alkenyl
-(C₁-C₁₀)alkynyl,
-R⁸,
R⁸-(C₁-C₈)alkyl,
R⁸-(C₂-C₈)alkenyl,
R⁸-(C₂-C₈)alkynyl,
-S-(C₁-C₁₀)-alkyl,
-SO₂-(C₁-C₁₀)-alkyl
-S-R⁸,
-SO₂-R⁸,
-CN
-Hal,
-O-(C₁-C₁₀)-alkyl, -NR⁶R⁷ wherein R⁶, R⁷ have the meaning defined above -O-R⁸, -OH
- R⁸: means an aryl group which may optionally be substituted by 1-3 hydroxy, halogen, C₁-C₅-alkyl, C₁-C₅-alkoxy, cyano, CF₃, nitro, COO(C₁-C₅-alkyl) or C(O)OCH₂-phenyl or a heteroaryl group whereby the heteroaryl group may contain 1-3 hetero atoms which may optionally be substituted by 1-3 alkyl groups, hydroxy, halogen, cyano or C₁-C₅-alkoxy groups,
- R⁹: means an hydrogen or a C₁-C₅-alkyl group
- R¹⁰: is a group 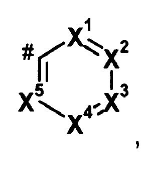 in which # denotes the point of attachment of the R¹⁰ group via a single bond,
- X¹, X², X³, X⁴, X⁵: independently of one another are nitrogen, or a group C- R¹¹
in which R¹¹ is selected from the group consisting of hydrogen, halogen, cyano, nitro, hydroxy, (C₁-C₅)-alkoxy, (C₁-C₅)-alkyl, (C₁- C₅)-halo-alkyl, (C₁-C₅)halo-alkoxy and COOR⁹,
and the R¹⁰ group contains a maximum of 3 nitrogen atoms in the ring,
and their salts, solvates or salts of solvates.

In another aspect the invention relates to compounds of general formula I in which
- R¹and R²: independently of one another, mean a hydrogen atom, a hydroxy group, a halogen atom, an optionally substituted (C₁-C₁₀)-alkyl group, an optionally substituted (C₁-C₁₀)-alkoxy group, a (C₁-C₁₀)- alkylthio group, a (C₁-C₅)-perfluoroalkyl group, a cyano group, a nitro group,
or R¹ and R²
together mean a group that is selected from the groups -O-(CH₂)ₚ- O-, -O-(CH₂)ₚ-CH₂-, -O-CH=CH-, -(CH₂)ₚ₊₂-, -NH-(CH₂)ₚ₊₁, -N(C₁- C₃-alkyl)-(CH₂)ₚ₊₁, and -NH-N=CH-,
whereby p = 1 or 2, and the terminal oxygen atoms and/or carbon atoms and/or nitrogen atoms are linked to directly adjacent ring-carbon atoms,
or NR⁶R⁷,
whereby R⁶ and R⁷, independently of one another, mean hydrogen, C₁-C₅-alkyl or (CO)-(C₁-C₅)-alkyl,
- R³: means a hydrogen atom, a hydroxy group, a halogen atom, a cyano group, an optionally substituted (C₁-C₁₀)-alkyl group, a (C₁- C₁₀)-alkoxy group, a (C₁-C₁₀)-alkylthio group, or a (C₁-C₅)- perfluoroalkyl group,
- R⁴: means a hydrogen, halogen, hydroxy, (C₁-C₅)-alkyl, (C₁-C₅)alkoxy,
- R⁵: means a group selected from
-(C₁-C₁₀)alkyl, which may be optionally partially or completely
halogenated
-(C₁-C₁₀)alkenyl
-(C₁-C₁₀)alkynyl,
-S-(C₁-C₁₀)-alkyl,
-SO₂-(C₁-C₁₀)-alkyl
-CN
-Hal,
-O-(C₁-C₁₀)-alkyl,
-NR⁶R⁷ wherein R⁶, R⁷ have the meaning defined above
-OH
- R¹⁰: is a group 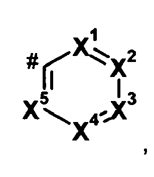 in which # denotes the point of attachment of the R¹⁰ group via a single bond,
- X¹, X², X³, X⁴, X⁵: independently of one another are nitrogen, or a group C- R¹¹
in which R¹¹ is selected from the group consisting of hydrogen, halogen, cyano, hydroxy, (C₁-C₅)-alkoxy, (C₁-C₅)-alkyl, (C₁-C₅)- halo-alkyl,
and the R¹⁰ group contains a maximum of 2 nitrogen atoms in the ring,
, and their salts, solvates or salts of solvates.

In another aspect the invention relates to compounds of general formula I in which
- R¹and R²: independently of one another, mean a hydrogen atom, a hydroxy
group, a halogen atom, an optionally substituted (C₁-C₁₀)-alkyl group, an optionally substituted (C₁-C₁₀)-alkoxy group, a (C₁-C₁₀)- alkylthio group, a (C₁-C₅)-perfluoroalkyl group, a cyano group, a nitro group,
or NR⁶R⁷,
whereby R⁶ and R⁷, independently of one another, mean hydrogen, C₁-C₅-alkyl or (CO)-(C₁-C₅)-alkyl,
- R³: means a hydrogen atom, a hydroxy group, a halogen atom, a cyano group, an optionally substituted (C₁-C₁₀)-alkyl group, a (C₁- C₁₀)-alkoxy group,
- R⁴: means a hydrogen, fluoro, (C₁-C₅)-alkyl,
- R⁵: means a group selected from -(C₁-C₁₀)alkyl, which may be optionally partially or completely halogenated -S-(C₁-C₁₀)-alkyl, -SO₂-(C₁-C₁₀)-alkyl -CN -Hal, -O-(C₁-C₁₀)-alkyl, -NR⁶R⁷ wherein R⁶, R⁷ have the meaning defined above -OH
- R¹⁰: is a group 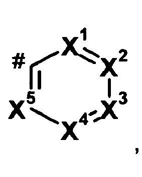 in which # denotes the point of attachment of the R¹⁰ group via a single bond,
- X¹, X², X³, X⁴, X⁵: independently of one another are nitrogen, or a group C- R¹¹ in which R¹¹ is selected from the group consisting of hydrogen, halogen, (C₁-C₅)-alkoxy, (C₁-C₅)-alkyl,
- and the R¹⁰: group contains a maximum of 2 nitrogen atoms in the ring, and their salts, solvates or salts of solvates.

Compounds of general formula I, wherein at least one of R¹, R² or R³ are different from hydrogen are one preferred embodiment of the invention.

In another embodiment two of R¹, R² or R³ according to claim 1 are different from hydrogen.

In one aspect of the invention the alkyl groups of the compounds of formula (I) have 1-5 carbon atoms.

In another aspect the alkyl groups of the compounds of formula (I) have 1-3 carbon atoms.

The quinolon ring of formula I can be substituted by a radical R⁴ selected from the group consisting of halogen, hydroxy, (C₁-C₅)-alkyl, (C₁-C₅)alkoxy, (C₁-C₅)-alkylthio, (C₁-C₅)-perfluoroalkyl, cyano, nitro, NR⁷R⁸ COOR⁹ (CO)NR⁷R⁸ or a (C₁-C₅-alkylene)-O-(CO)-(C₁-C₅)alkyl group, preferably R⁴ is selected from the group C₁-C₃-alkyl, C₁-C₃-alkoxy, hydroxy, halogen.

In another aspect of the invention R⁴ is selected from the group hydrogen, C₁-C₃-alkyl, halogen, hydroxy, preferably from hydrogen or halogen, more preferably from hydrogen, chlorine or fluorine.

Another subject of the invention are compounds according to formula 1 wherein R⁴ is hydrogen or fluorine.

Yet another subject of the invention are compounds according to formula 1 wherein R⁴ is fluorine.

More particularly are compounds according to formula 1 wherein R⁴ is a 7-fluoro-substituent or hydrogen and at least one of R¹, R² and R³ is selected from chlorine, fluorine, methoxy, R⁵ is selected from S-CH₂-CH₃, -O-CH₂-CH₃, - S-CH₃, -O-CH₃ -, -OH and dimethylamino.

One aspect of the invention are compounds of general formula I, wherein the phenyl group is substituted with 1-3 of the same or different substituents R¹, R² and R³. R¹ and R² are independently of one another, mean a hydrogen atom, a hydroxy group, a halogen atom, an optionally substituted (C₁-C₁₀)-alkyl group, an optionally substituted (C₁-C₁₀)-alkoxy group, a (C₁-C₁₀)-alkylthio group, a (C₁-C₅)-perfluoroalkyl group, a cyano group, a nitro group, or R¹ and R² together mean a group that is selected from the groups -O-(CH₂)ₚ-O-, -O-(CH₂)ₚ-CH₂-, -O-CH=CH-, -(CH₂)ₚ₊₂-, -NH-(CH₂)ₚ₊₁, -N(C₁-C₃-alkyl)-(CH₂)ₚ₊₁. and -NH-N=CH-, whereby p = 1 or 2, and the terminal oxygen atoms and/or carbon atoms and/or nitrogen atoms are linked to directly adjacent ring-carbon atoms, or R¹ and R² are NR⁶R⁷, whereby R⁶ and R⁷, independently of one another, mean hydrogen, C₁-C₅-alkyl or (CO)-(C₁-C₅)-alkyl. The third substituent R³ means a hydrogen atom, a hydroxy group, a halogen atom, a cyano group, an optionally substituted (C₁-C₁₀)-alkyl group, a (C₁-C₁₀)-alkoxy group, a (C₁-C₁₀)-alkylthio group, or a (C₁-C₅)-perfluoroalkyl group.

In another aspect any other phenyl group may be substituted by a group selected from ,C₁-C₃-alkoxy, hydroxy, and halogen, in particular methoxy, hydroxy, fluorine, chlorine, or bromine.

In another aspect of the invention R⁵ selected from -(C₁-C₁₀)-alkyl, which may be optionally partially or completely halogenated, -(C₁-C₁₀)alkenyl
-(C₁-C₁₀)alkynyl,
R⁸, R⁸-(C₁-C₈)alkyl, R⁸-(C₂-C₈)alkenyl, R⁸-(C₂-C₈)alkynyl, -S-(C₁-C₁₀)alkyl, -SO₂-(C₁-C₁₀)alkyl-S-R⁸, -SO₂-R⁸, -CN, -Hal, -O-(C₁-C₁₀)alkyl, - NR⁶R⁷ (wherein R⁶, R⁷ have the meaning defined above), -O-R⁸ and -OH.

In yet another aspect R⁵ is selected from the group consisting of -(C₁-C₁₀)-alkyl, which may be optionally partially or completely halogenated, -S-(C₁-C₁₀)-alkyl, -SO₂-(C₁-C₁₀)-alkyl, -CN, -Hal, -O-(C₁-C₁₀)-alkyl, -NR⁶R⁷ (wherein R⁶, R⁷ have the meaning defined above), -OH, preferably R⁵ is -OH, Cl, -S-CH₃, -S-CH₂-CH₃, -S-CH₂-CH₂-CH₃, -O-CH₃, -O-CH₂-CH₃, -O-CH₂-CH₂-CH₃, R⁵ is most preferably -OH, -O-CH₃, or -O-CH₂-CH₃.

In yet another aspect R⁵ is selected from the group consisting of -(C₁-C₁₀)-alkyl, -S-(C₁-C₁₀)-alkyl, -SO₂-(C₁-C₁₀)-alkyl, -O-(C₁-C₁₀)-alkyl.
More particularly are compounds according to formula 1 wherein R¹⁰ is a group 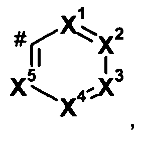 in which # denotes the point of attachment of the R¹⁰ group via a single bond,
- X¹, X², X³, X⁴, X⁵: independently of one another are nitrogen, or a group C- R¹¹ in which R¹¹ is (C₁-C₅)-alkoxy, (C₁-C₅)-alkyl, halogen and the maximum of one or two nitrogen atoms in the ring,

Even more particularly are compounds according to formula 1 wherein R¹⁰ is a group 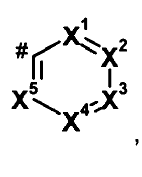 in which # denotes the point of attachment of the R¹⁰ group via a single bond,
- X¹, X³, X⁵: are a C- R¹¹ Group in which R¹¹ is (C₁-C₅)-alkoxy, (C₁-C₅)-alkyl, halogen and X², X⁴ are independently of one another are nitrogen or a C- R¹¹ Group in which R¹¹ is (C₁-C₅)-alkoxy, (C₁-C₅)-alkyl, halogen, and the number of nitrogen atoms in the ring are one or two.

One aspect of the invention are compounds of formula I
5-{[1-(2-Chloro-3-fluoro-4-methoxyphenyl)-3,3,3-trifluoro-2-hydroxy-2-(methoxymethyl)propyl]amino}-7-fluoro-1-(4-fluorophenyl)quinolin-2-one
5-{[1-(2-Chloro-3-fluoro-4-methoxyphenyl)-3,3,3-trifluoro-2-hydroxy-2-(methoxymethyl)propyl]amino}-7-fluoro-1-(6-fluoropyridin-3yl)quinolin-2-one
5-{[1-(2-Chloro-3-fluoro-4-methoxyphenyl)-3,3,3-trifluoro-2-hydroxy-2-({methylsulfanyl}methyl)propyl]amino}-7-fluoro-1-(4-fluorophenyl)quinolin-2-one
5-{[1-(2-Chloro-3-fluoro-4-methoxyphenyl)-3,3,3-trifluoro-2-hydroxy-2-({ethylsulfanyl}methyl)propyl]amino}-7-fluoro-1-(6-fluoropyridin-3y)quinolin-2-one
5-{[1-(2-Chloro-3-fluoro-4-methoxyphenyl)-3,3,3-trifluoro-2-hydroxy-2-(methoxymethyl)propyl]amino}-7-fluoro-1-(2-methoxypyrimidin-5yl)quinolin-2-one
5-{[1-(2-Chloro-3-fluoro-4-methoxyphenyl)-3,3,3-trifluoro-2-hydroxy-2-({ethylsulfanyl}methyl)propyl]amino}-7-fluoro-1-(6-metoxypyridin-3yl)quinolin-2-one
5-{[1-(2-Chloro-3-fluoro-4-methoxyphenyl)-2-({dimethylamino}methyl)-3,3,3-trifluoro-2-hydroxypropyl]amino}-7-fluoro-1-(4-fluorophenyl)quinolin-2-one
5-{[1-(2-Chloro-3-fluoro-4-methoxyphenyl)-3,3,3-trifluoro-2-hydroxy-2-(hydroxymethyl)propyl]amino}-7-fluoro-1-(6-fluoropyridin-3yl)quinolin-2-one
5-{[1-(4-Chloro-3-fluoro-2-methoxyphenyl)-3,3,3-trifluoro-2-hydroxy-2-(methoxymethyl)propyl]amino}-1-(2-fluoropyrimidin-5-yl)-1*H*-quinolin-2-one
5-{[2-(Ethoxymethyl)-1-(3-fluoro-4-methoxyphenyl)-3,3,3-trifluoro-2-hydroxypropyl]amino}-1-(pyrimidin-4-yl)-quinolin-2-one-1H--quinolin-2-one
1-(4-Fluorophenyl)-5-{[3,3,3-trifluoro-1-(3-fluoro-4-methoxy-2-methylphenyl)-2-hydroxy-2-({methylsulfonyl}methyl)propyl]amino}-1*H*-quinolin-2-one
5-{[1-(2-Chloro-3-fluoro-4-methoxyphenyl)-2-({ethylsulfonyl}methyl)-3,3,3-trifluoro-2-hydroxypropyl]amino}-1-(4-fluorophenyl)-7-methyl-1*H*-quinolin-2-one
5-{[1-(2-Chloro-4-ethylphenyl)-3,3,3-trifluoro-2-hydroxy-2-(methoxymethyl)propyl]amino}-1-(2-methoxypyrimidin-5-yl)-1*H*-quinolin-2-one
5-{[1-(4-Chloro-3-fluoro-2-hydroxyphenyl)-3,3,3-trifluoro-2-hydroxy-2-(hydroxymethyl)propyl]amino}-7-fluoro-1-(6-fluoropyridin-3yl)-1*H*-quinolin-2-one
5-{[1-(2-Chloro-3-fluoro-4-methoxyphenyl)-2-({dimethylamino}methyl)-3,3,3-trifluoro-2-hydroxypropyl]amino}-7-fluoro-1-(6-fluoropyridin-2-yl)-1*H*-quinolin-2-one
5-{[2-({Ethylsulfanyl}methyl)-1-(3-fluoro-4-methoxyphenyl)-3,3,3-trifluoro-2-hydroxypropyl]amino}-1-(2-methylpyrimidin-5-yl)-1*H*-quinolin-2-one
5-{[-(2-Chloro-4-methylphenyl)-3,3,3-trifluoro-2-hydroxy-2-(methoxymethyl)propyl]amino}-1-(2-methylpyridin-4-yl)-1*H*-quinolin-2-one
7-Fluoro-1-(6-fluoropyridin-3-yl)-5-{[3,3,3-trifluoro-1-(3-fluoro-4-methylphenyl)-2-hydroxy-2-(hydroxymethyl)propyl]amino}-1*H*-quinolin-2-one

One aspect of the invention are the subcombinations of all residues as disclosed in examples 1-18.

In another aspect the example compounds 1, 2, 5, 6, and 7 are preferred.

In one aspect R¹⁰ is not phenyl, in another aspect R¹⁰ is not naphthyl, in yet another aspect R¹⁰ is not phenyl or naphthyl.

An aspect of the invention is a method of treating a glucocorticoid receptor mediated disease state in a mammal, which comprises administering to a mammal in need of such treatment an effective amount of a compound of formula (I), or a pharmaceutically acceptable salt thereof.

In addition, the invention relates to the use of the compounds of general formula I for the production of pharmaceutical agents as well as their use for the production of pharmaceutical agents for treating inflammatory diseases.

### Definitions

Unless otherwise notifed the term "alkyl" refers to a straight or branched, substituted or unsubstituted chain. For example, the term propyl comprises *ⁿ-*propyl and *^{iso}*-propyl, the term butyl comprises *ⁿ*-butyl, *^{iso}*-butyl and *^{tert.}*-butyl.

The alkyl groups can be straight-chain or branched and stand e.g. for a methyl, ethyl, n-propyl, iso-propyl, n-butyl, iso-butyl, tert-butyl or n-pentyl group, or a 2,2-dimethylpropyl, 2-methylbutyl or 3-methylbutyl group. One aspect are (C₁-C₃)alkyl groups. A methyl or ethyl group is preferred. They can optionally be substituted by 1-3 hydroxy groups, cyano groups, halogen, 1-3 C₁-C₅-alkoxy groups, and/or 1-3 COO(C₁-C₁₀-alkyl or benzyl) groups. Preferred are hydroxy groups. The total number of substituents depends on the number of carbon atoms of the chain. Usually the number of substituents does not exceed the number of carbon atoms except for halogen which leads at a maximum number of substituents to e.g. perfluorated alkyl groups.

For a partially or completely fluorinated C₁-C₃-alkyl group, the following partially or completely fluorinated groups are considered: fluoromethyl, difluoromethyl, trifluoromethyl, fluoroethyl, 1,1-difluoroethyl, 1,2-difluoroethyl, 1,1,1-trifluoroethyl, tetrafluoroethyl, and pentafluoroethyl. Of the latter, the trifluoromethyl group or the pentafluoroethyl group is preferred.

The C₁-C₅-alkoxy groups in R¹, R², R³ and R⁵ can be straight-chain or branched and stand for a methoxy, ethoxy, n-propoxy, iso-propoxy, n-butoxy, iso-butoxy, tert-butoxy or n-pentoxy, 2,2-dimethylpropoxy, 2-methylbutoxy or 3-methylbutoxy group. A methoxy or ethoxy group is preferred. They can optionally be substituted by C₁-C₅-alkyl groups, cyano groups or halogen

The C₁-C₅-alkylthio groups can be straight-chain or branched and stand for a methylthio, ethylthio, n-propylthio, iso-propylthio, n-butylthio, iso-butylthio, tert-butylthio or n-pentylthio, 2,2-dimethylpropylthio, 2-methylbutylthio or 3-methylbutylthio group. A methylthio or ethylthio group is preferred.

The term halogen atom, Hal or halogen means a fluorine, chlorine, bromine or iodine atom. Preferred is a fluorine, chlorine or bromine atom.

The NR⁶R⁷ group includes, for example, NH₂, N(H)CH₃, N(CH₃)₂, N(H)(CO)CH₃, N(CH₃)(CO)CH₃, N[(CO)CH₃]₂, N(H)CO₂CH₃, N(CH₃)CO₂CH₃, or N(CO₂CH₃)₂.

The term C₂-C₈-alkenyl is a straight or branched, substituted or unsubstituted, chain including isomers having an E- or Z-configurated double bond such as e.g. vinyl, propen-1-yl, propen-2-yl (Allyl), but-1-en-1-yl, but-1-en-2-yl, but-2-en-1-yl, but-2-en-2-yl, 2-methyl-prop-2-en-1-yl, 2-methyl-prop-1-en-1-yl, but-1-en-3-yl, but-3-en-1-yl. If the alkenyl residue is placed between two other moieties the term alkenyl means alkenylene such as e.g. vinylene, propen-1-ylene, propen-2-ylene (Allylen), but-1-en-1-ylene, but-1-en-2-ylene, but-2-en-1-ylene, but-2-en-2-ylene, 2-methyl-prop-2-en-1-ylene, 2-methyl-prop-1-en-1-ylene, but-1-en-3-ylen, but-3-en-1-ylene.

The term C₂-C₈-alkynyl stands for a straight or branched chain e,g,-C≡CH, -CH₂-C≡CH, -C≡C-CH₃, -CH(CH₃)-C≡CH, -C≡C-CH₂(CH₃), -C(CH₃)₂-C≡CH, -C≡C-CH(CH₃)₂, -CH(CH₃)-C≡C-CH₃, , -CH₂-C≡C-CH₂(CH₃) or, if the alkynyl residue is placed between two other moieties the term alkynyl means alkynylene such as e.g. -C≡C-, -CH₂-C≡C-, -C≡C-CH₂-, -CH(CH₃)-C≡C-, -C≡C-CH(CH₃)-, -C(CH₃)₂-C≡C-, -C≡C-C-(CH₃)₂-, -CH(CH₃)-C≡C-CH₂-, -CH₂-C≡C-CH (CH₃)-.

The term C₃-C₇-cycloalkyl means a substituted or unsubstituted group selected from cyclopropyl, cyclobutyl, cyclopentyl, cyclohexyl, cycloheptyl. The possible substitutents may be selected from hydroxy, halogen, (C₁-C₅)-alkyl, (C₁-C₅)-alkoxy, NR⁴R⁵, COO(C₁-C₅)-alkyl, CHO, cyano.

The term C₃-C₇-cycloalkyl-(C₁-C₁₀)-alkyl- means e.g. -(CH₂)-cycloalkyl, -(C₂H₄)-cycloalkyl, -(C₃H₆)-cycloalkyl, -(C₄H₈)-cycloalkyl, -(C₅H₁₀)-cycloalkyl whereby the cycloylkyl stand for e.g. cyclopropyl, cyclobutyl, cyclopentyl, cyclohexyl, cycloheptyl.

The term C₃-C₇-cycloalkyl-(C₂-C₈)-alkenyl means e.g. -(CH=CH)-cycloalkyl, -[C(CH₃)=CH]-cycloalkyl, -[CH=C(CH₃)]-cycloalkyl, -(CH=CH-CH₂)-cycloalkyl, -(CH₂-CH=CH)-cycloalkyl, -(CH=CH-CH₂-CH₂)-cycloalkyl, -(CH₂-CH=CH-CH₂)-cycloalkyl, -(CH₂-CH₂-CH=CH)-cycloalkyl, -(C(CH₃)=CH-CH₂)-cycloalkyl, -(CH=C(CH₃)-CH₂)-cycloalkyl whereby the term cycloalkyl is defined above..

The term heterocyclyl means e.g. piperidinyl-, morpholinyl-, thiomorpholinyl-, piperazinyl-, tetrahydrofuranyl-, tetrahydrothienyl-, imidazolidinyl- or pyrrolidinyl- whereby the heterocyclyl group may be bound via any possible ring atom..

The heterocyclyl group may be substituted by C₁-C₅-alkyl (optionally substituted), hydroxy-, C₁-C₅-alkoxy-, NR⁴R⁵-, halogen, cyano-, COOR⁸-, CHO-. If possible these substitutens may also be bound to one of the free nitrogen atoms if any. N-oxides are also included in the definition.

The term heterocyclyl-(C₁-C₁₀)-alkenyl- means an alkylene group as defined above which is connected to the heterocyclyl group which also is already defined above.

The term heterocyclyl-(C₂-C₈)-alkenyl- means an alkylenylene group as defined above which is connected to the heterocyclyl group which also is already defined above.

The term aryl in the sense of the invention means aromatic or partially aromatic carbocyclic rings having 6 to 14 carbon atoms, e.g. phenyl and which may also may have a condensed a second or third ring such as e.g. napthyl or anthranyl. Further examples are phenyl, naphthyl, tetralinyl, anthranyl, benzoxazinone, dihydroindolone, indanyl, and indenyl.

The aryl groups may be substituted at any position leading to a stable molecule by one or several substitutents, e.g. 1-3 substitutents, such as e.g. hydroxy, halogen, C₁-C₅-alkyl, C₁-C₅-alkoxy, cyano, CF₃, nitro, COO(C₁-C₅-alkyl or benzyl) or a heteroaryl group , preferably by 1-3 C₁-C₅-alkyl groups, hydroxyl, halogen, cyano or C₁-C₅-alkoxy. The optionally substituted phenyl group is one aspect of the invention. Yet another aspect are the compounds of formula I whereby R⁸ is not phenyl.

The term heteroaryl means an aromatic ring system having 1-3 heteroatoms selected from nitrogen, oxygen or sulfur, for five membered rings the maximum number of heteroatoms is three whereby only two oxygen or sulfur atoms are allowed provided that these two are not directly bound to each other.

Possible heteroaryl rings are e.g. thienyl, furanyl, pyrrolyl, oxazolyl, thiazolyl, imidazolyl, pyrazolyl, isoxazolyl, isothiazolyl, oxadiazolyl, triazolyl, thiadiazolyl, benzofuranyl, benzothienyl, benzothiazol, benzoxazolyl, benzimidazolyl, indazolyl, indolyl, isoindolyl, pyridyl, pyridazinyl, pyrimidinyl, pyrazinyl, triazinyl, azaindolizinyl-,benzopyridyl, benzopyridazinyl, benzopyrimidinyl, benzopyrazinyl, benzotriazinyl, quinolyl, isoquinolyl, phthalidyl-, thiophthalidyl, indolonyl-, dihydroindolonyl-, isoindolonyl-, dihydroisoindolonyl-, benzofuranyl-or benzimidazolyl.

The compounds of the present invention can exist in stereoisomeric forms such as enantiomers of diastereoisomers depending on their structure and residues as defined in formula I. In one aspect of the invention therefore all these enantiomers, diastereoisomers or mixtures thereof are encompassed. The isolation of enantiomerically or diastereomerically pure isomers can be done by methods of the state of the art, e.g. using column chromatography with a chiral solid phase.

Should it be possible that the compounds of the invention also exist in tautomeric forms these are also an aspect of the present invention.

In one aspect of the invention all compounds defined in formula I as well as their salts, solvates and solvates of salts are encompassed,. especially the salts, solvates and salts of solvates of the compounds disclosed in the examples are one aspect of the invention as long as the disclosed compounds themselves are not already salts, solvates or solvates of the salts.

Salts in the sense of the present invention are not only physiologically unobjectable salts but also salts which might be objectable for pharmaceutical use but which are useful e.g. during the process of isolation or purification.

The term physiologically unobjectable salts includes addition salts of mineral acids, carbonic acids, sulfonic acids, e.g. salts of hydrochloric acid, hydrobromic acid, sulfuric acid, nitric acid, phosphoric acid, methanesulfonic acid, ethanesulfonic acid, toluolsulfonic acid, benzenesulfonic acid, naphthalinesulfonic acid, acetic acid, trifluoroacetic acid, propionic acid, lactic acid, tartaric acid, malic acid, citric acid, fumaric acid, pivalic acid, maleic acid, succinic acid and benzoic acid.

In addition the term physiologically unobjectable salts includes salts of commonly suitable bases, e.g. salts of alkalimetall (e.g.. sodium- and potassium salts), alkaline earth salts (e.g. calcium- and magnesium salts) and ammonium salts, derivatized from NH₃ or organic amines with 1 to 16 carbon atoms, e.g. ethylamine, diethylamine, triethylamine, ethyldiisopropylamine, monoethanolamine, diethanolamine, triethanolamine, dicyclohexylamine, dimethylaminoethanol, prokaine, dibenzylamine, N-methylmorpholin, arginin, lysin, ethylendiamine and N-methylpiperidin.

Solvates in the sense of the invention are such forms of the compounds of the present combinations which build complexes by coordination of solvent molecules in a liquid or a solid phase. Hydrates are special forms of a solvate wherein water molecules are coordinated.

Salts in the sense of the present invention are not only physiologically unobjectable salts but also salts which might be objectable for pharmaceutical use but which are useful e.g. during the process of isolation or purification.

The compounds can be produced by the two processes that are described below (c).

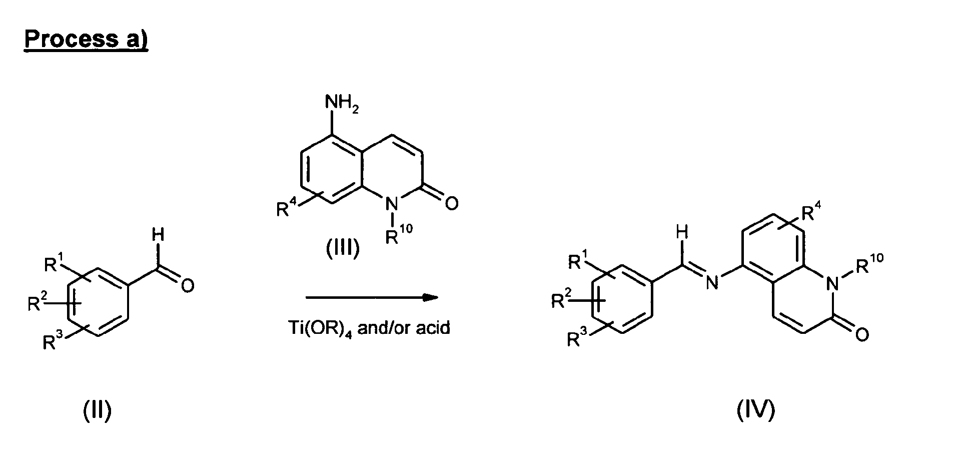

Benzaldehydes (commercially available or readily available by methods according to application EP 1878717 A1 .of type (II) can be condensed with substituted aminoquinolones of type (III) to imines of type (IV) using Lewis acids, preferably titanium alcoholates Ti(OR)₄ wherein R is C₁-C₄-alkyl, such as e.g. tetraethyl orthotitanate or tetra *tert*. butyl orthotitanate and/or acidic conditions, e.g. organic acids such as acetic acid as reagents. Suitable solvents are e.g. toluene, 1,4-dioxane or mixtures thereof.

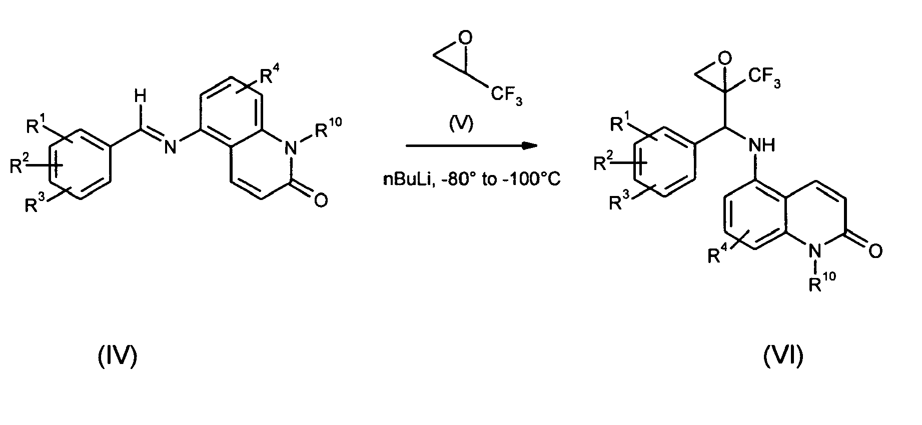

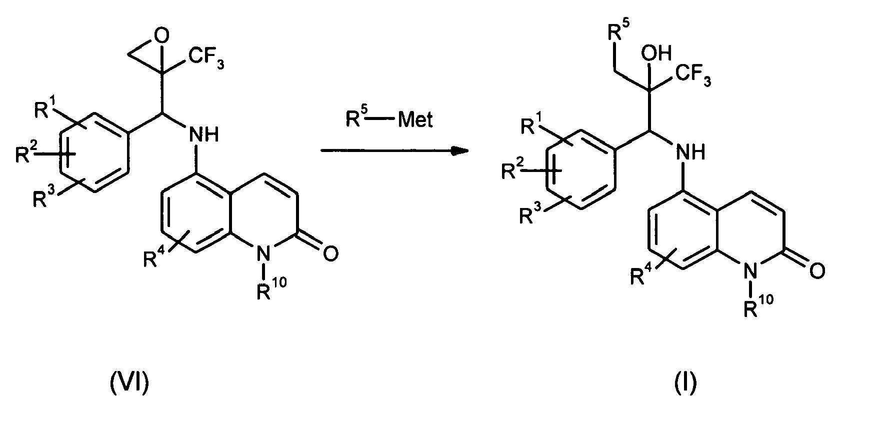

Imines of type (IV) are treated at low temperatures of -80° to -100°C with the lithiated epoxide (V) to yield compounds of type (VI). Suitable solvents are e.g. tetrahydrofurane, hexane, diethylether or mixtures thereof. The epoxides (VI) can be opened by nucleophiles of type R⁵-Met to deliver compound (I). Met means metal and includes alkalimetals e.g. caesium, potassium, sodium or lithium, alkaline earth metals such as e.g. magnesium and calcium, or aluminium, copper, silicon or tin (Sn) which bind the nucleophilic residue R⁵ of R⁵-Met depending on their valence and according to the knowledge of a person with ordinary skill. The resulting possible nucleophilic reagents R⁵-Met are e.g. alkylcuprates, vinylcuprates, thioles, allylsilanes, vinylsilanes, vinylstannanes, grignard compounds whereby R⁵ is defined as in claim 1, which react in the presence of Lewis acids like e.g. boron trifluoride or trimethylaluminium, aluminium trichloride, titanium tetrachloride or isopropyl magnesium chloride. Suitable solvents are e.g. diethylether, dimethylformamide, tetrahydrofurane. The epoxides (VI) can also be opened directly by cyanides, amines, alcoholates, thioalcoholates, halogenides and even water or caesium carbonate/water in the presence of bases or strong protic acids. Suitable bases in the sense of the invention are e.g. caesium carbonate potassium carbonate or sodium hydroxide.

Suitable strong protic acids are e.g. perchloric acid, hydrochloric acid or hydrobromic acid.

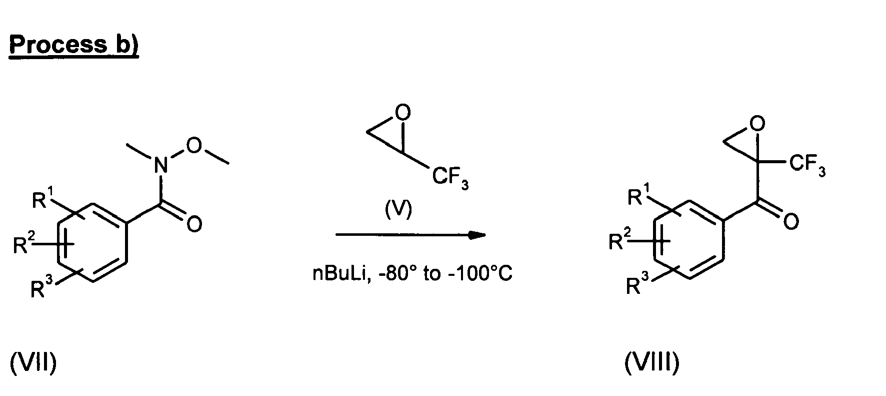

Methoxymethylamides of type (VII) obtained by a process according to J: C: Woo et al. J.Org.Chem. 2004, 69, 8984-8986 or J. Singh al. J.Prakt.Chem. 2000, 342, 340-347 are treated at low temperatures of -80°to -100°C with the lithiated epoxide (V) to yield compounds of type (VIII).

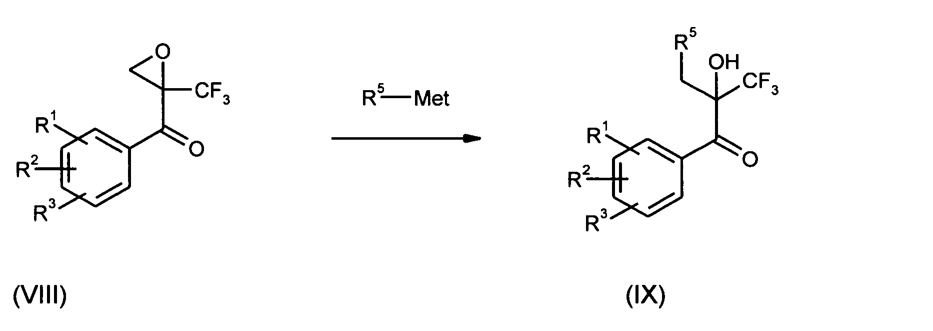

The epoxides (VIII) can be opened by nucleophils of type R⁵-Met [see also process a)] to deliver compound (IX). Possible nucleophiles are alkylcuprates, vinylcuprates, thioles, allylsilanes, vinylsilanes, vinylstannanes, grignard compounds, in the presence of Lewis acids like boron trifluoride or trimethylaluminium, aluminium trichloride, or directly by cyanides, amines, alcohols, thioalcoholes, halogenides and water in the presence of bases or strong protic acids.

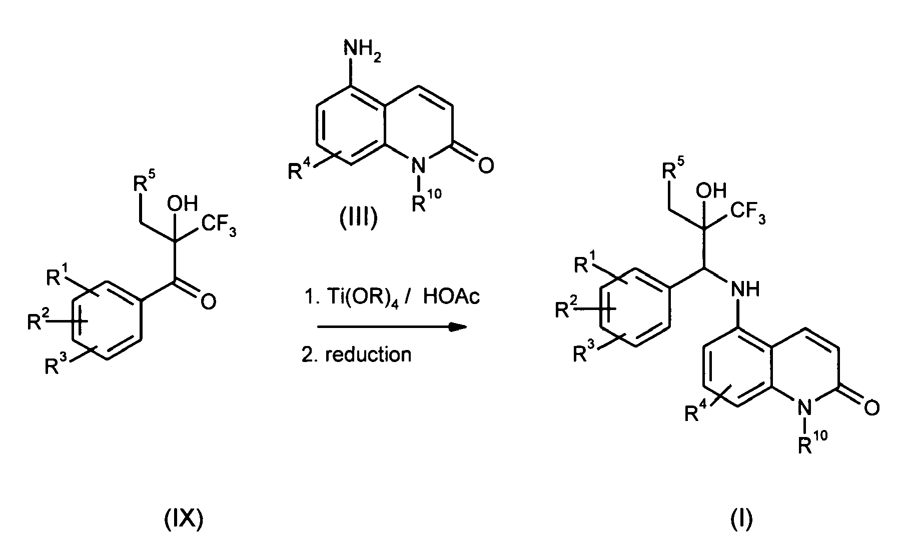

Ketones of type (IX) can be condensed with substituted aminoquinolones of type (III) to imines and subsequently or simultaneously reduced to the aminoalcohol I by a reductive amination using complex hydrides like e.g. sodium borohydride or lithium aluminium hydride (Katritzky et al. J.Org.Chem. 1995, 60, 7631-7640) or hydrogen in the presence of catalytic amounts of palladium or platinum or by application of an asymmetric organocatlytic transfer hydrogenation (List et al. Angew. Chem. 2005, 117, 7590-7593).

This processes described above can be perfomed enantioselectively by use of commercially available enantiopure epoxide of formula (V) to yield enantiopure compounds of formula (VI), (VIII), (IX) and (I). The last reductive step of b)(IX → I, second step) can be performed in a diastereoselective manner to yield enantiopure compound I when enantiopure compound IX is used as starting material.

Alternatively during the process of the production of the compounds of formula I at different stages purification for obtaining enantiomerically or diastereomerically pure intermediates my be performed e.g. intermediates of formula VI, VIII, IX can be purified at the step when they are obtained or compounds of formula I can be purified to obtain enantiomerically or diastereomerically pure end products after the complete reaction cascade. Examples for methods for obtaining enantiopure (enantiomerically pure) compounds are described below. The separation of optical isomers can be performed by separation of one or more of the intermediates and/or separation of the end products. Usually separation of intermediates and separation of end products are alternatives as long as no racemisation had taken place during the production process.

If the compounds according to the invention are present as racemic mixtures, they can be separated into pure, optically active forms according to the methods of racemate separation that are familiar to one skilled in the art. For example, the racemic mixtures can be separated by chromatography on an even optically active carrier material (CHIRALPAK AD^{®}) into the pure isomers. It is also possible to use chiral auxiliary agents as optically pure acids. For that purpose the free hydroxy group is esterified to yield a racemic compound of general formula I with an optically active acid and to separate the diastereoisomeric esters that are obtained by fractionated crystallization or by chromatography, and to saponify the separated esters in each case to the optically pure isomers. As an optically active acid, for example, mandelic acid, camphorsulfonic acid or tartaric acid can be used.

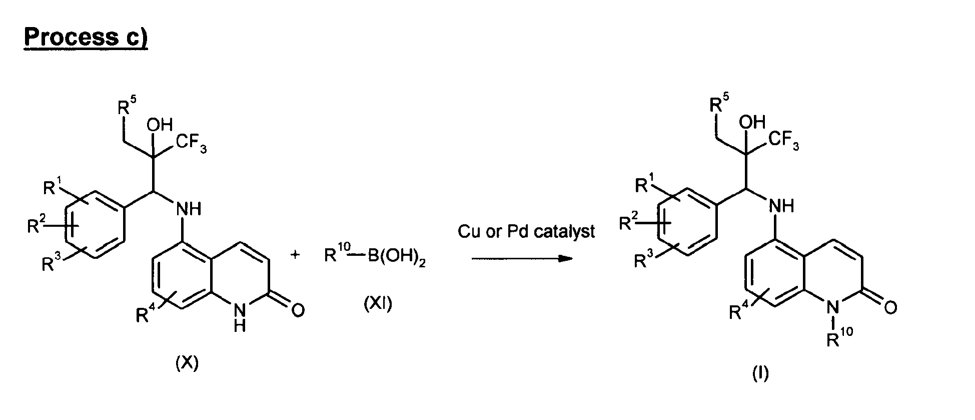

An quinoline of general formula (X) which is known from EP 1878717 A1, in which , R¹ , R², R³, R⁴ and R⁵ have the meanings that are indicated for formula (I), is converted with an arylboronic acid of the general formula (XI), in which under R¹⁰ has the meaning that is indicated for formula (I), under catalysis of copper or palladium (P. Lam et al. Syn. Lett. 2000; 5, 674 - 76; H. Sano et al, Biorg. Med. Chem. 2005, 13, 3079-91) to compounds of the general formula (I) according to the invention.

The binding of the substances to the glucocorticoid receptor (GR) and other steroid hormone receptors (mineral corticoid receptor (MR), progesterone receptor (PR) and androgen receptor (AR)) is examined with the aid of recombinantly produced receptors. Cytosol preparations of Sf9 cells, which had been infected with recombinant baculoviruses, which code for the GR, are used for the binding studies.

As an essential, molecular mechanism for the anti-inflammatory action of glucocorticoids, the GR-mediated inhibition of the transcription of cytokines, adhesion molecules, enzymes and other pro-inflammatory factors is considered. This inhibition is produced by an interaction of the GR with other transcription factors, e.g., AP-1 and NF-kappa-B (for a survey, see Cato, A. C. B., and Wade, E., BioEssays 18, 371-378, 1996).

The compounds of general formula I according to the invention inhibit the secretion of cytokine IL-8 into the human monocyte cell line THP-1 that is triggered by lipopolysaccharide (LPS). The concentration of the cytokines was determined in the supernatant by means of commercially available ELISA kits.

The compound from Examples 1 and 2 showed an inhibition IC₅₀(IL8) = 0.79 nmol, and IC₅₀(IL8) = 0.31 nmol with efficacies of 99%, and 107% respectively in comparison with dexamethasone as reference. IC₅₀(GR) 7.7 nM (example 1) and IC₅₀(GR) 6.3 nM (example 2) were measured

For comparison the examples 3 of EP 1878717 A1.showed an inhibition IC₅₀(IL8) = 25 nmol. Example 18 of WO 2005/035518 A1 showed an inhibition IC₅₀(IL8) = 2,8 nmol with efficacy of 96% in comparison with dexamethasone as reference. IC₅₀(GR) 64 nM (example 3) and IC₅₀(GR) 60 nM (example 18) were measured.

The anti-inflammatory action of the compounds of general formula I was tested in the animal experiment by tests in the croton oil-induced inflammation in rats and mice (J. Exp. Med. 1995, 182, 99-108). To this end, croton oil in ethanolic solution was applied topically to the animals' ears. The test substances were also applied topically or systemically at the same time or two hours before the croton oil. After 16-24 hours, the ear weight was measured as a yardstick for inflammatory edema, the peroxidase activity as a yardstick for the invasions of granulocytes, and the elastase activity as a yardstick for the invasion of neutrophilic granulocytes. In this test, the compounds of general formula I inhibit the three above-mentioned inflammation parameters both after topical administration and after systemic administration.

One of the most frequent undesirable actions of a glucocorticoid therapy is the so-called "steroid diabetes" [cf., Hatz, H. J., Glucocorticoide: Immunologische Grundlagen, Pharmakologie und Therapierichtlinien [Glucocorticoids: Immunological Bases, Pharmacology and Therapy Guidelines], Wissenschaftliche Verlagsgesellschaft mbH, Stuttgart, 1998]. The reason for this is the stimulation of gluconeogenesis in the liver by induction of the enzymes responsible in this respect and by free amino acids, which are produced from the degradation of proteins (catabolic action of glucocorticoids). A key enzyme of the catabolic metabolism in the liver is tyrosinamino transferase (TAT). The activity of this enzyme can be determined from liver homogenates by photometry and represents a good measurement of the undesirable metabolic actions of glucocorticoids. To measure the TAT induction, the animals are sacrificed 8 hours after the test substances are administered, the livers are removed, and the TAT activity is measured in the homogenate. In this test, at doses wherein they have an anti-inflammatory action, the compounds of general formula I induce little or no tyrosinamino transferase.

Because of their anti-inflammatory and, in addition, anti-allergic, immunosuppressive and antiproliferative action, the compounds of general formula I according to the invention can be used as medications for treatment or prophylaxis of the following pathologic conditions in mammals and humans: In this case, the term "DISEASE" stands for the following indications:
(i) Lung diseases, which coincide with inflammatory, allergic and/or proliferative processes:
   - Chronic, obstructive lung diseases of any origin, primarily bronchial asthma
   - Bronchitis of different origins
   - Adult respiratory distress syndrome (ARDS), acute respiratory distress syndrome
   - Bronchiectases
   - All forms of restrictive lung diseases, primarily allergic alveolitis,
   - All forms of pulmonary edema, primarily toxic pulmonary edema; e.g., radiogenic pneumonitis
   - Sarcoidoses and granulomatoses, especially Boeck's disease
(ii) Rheumatic diseases/autoimmune diseases/joint diseases, which coincide with inflammatory, allergic and/or proliferative processes:
   - All forms of rheumatic diseases, especially rheumatoid arthritis, acute rheumatic fever, polymyalgia rheumatica, Behçet's disease
   - Reactive arthritis
   - Inflammatory soft-tissue diseases of other origins
   - Arthritic symptoms in the case of degenerative joint diseases (arthroses)
   - Traumatic arthritides
   - Vitiligo
   - Collagenoses of any origin, e.g., systemic lupus erythematodes, sclerodermia, polymyositis, dermatomyositis, Sjögren's syndrome, Still's syndrome, Felty's syndrome
   - Sarcoidoses and granulomatoses
   - Soft-tissue rheumatism
(iii) Allergies or pseudoallergic diseases, which coincide with inflammatory and/or proliferative processes:
   - All forms of allergic reactions, e.g., Quincke's edema, hay fever, insect bites, allergic reactions to pharmaceutical agents, blood derivatives, contrast media, etc., anaphylactic shock, urticaria, allergic and irritative contact dermatitis, allergic vascular diseases
   - Allergic vasculitis
(iv) Vascular inflammations (vasculitides)
   - Panarteritis nodosa, temporal arteritis, erythema nodosum
   - Polyarteris nodosa
   - Wegner's granulomatosis
   - Giant-cell arteritis
(v) Dermatological diseases, which coincide with inflammatory, allergic and/or proliferative processes:
   - Atopic dermatitis (primarily in children)
   - All forms of eczema, such as, e.g., atopic eczema (primarily in children)
   - Rashes of any origin or dermatoses
   - Psoriasis and parapsoriasis groups
   - Pityriasis rubra pilaris
   - Erythematous diseases, triggered by different noxae, e.g., radiation, chemicals, burns, etc.
   - Bullous dermatoses, such as, e.g., autoimmune pemphigus vulgaris, bullous pemphigoid
   - Diseases of the lichenoid group,
   - Pruritis (e.g., of allergic origin)
   - Seborrheal eczema
   - Rosacea group
   - Erythema exudativum multiforme
   - Balanitis
   - Vulvitis
   - Manifestation of vascular diseases
   - Hair loss such as alopecia areata
   - Cutaneous lymphoma
(vi) Kidney diseases, which coincide with inflammatory, allergic and/or proliferative processes:
   - Nephrotic syndrome
   - All nephritides, e.g., glomerulonephritis
(vii) Liver diseases, which coincide with inflammatory, allergic and/or proliferative processes:
   - Acute liver cell decomposition
   - Acute hepatitis of different origins, e.g., viral, toxic, pharmaceutical agent-induced
   - Chronic aggressive hepatitis and/or chronic intermittent hepatitis
(viii) Gastrointestinal diseases, which coincide with inflammatory, allergic and/or proliferative processes:
   - Regional enteritis (Crohn's disease)
   - Colitis ulcerosa
   - Gastritis
   - Reflux esophagitis
   - Ulcerative colitis of other origins, e.g., native sprue
(ix) Proctologic diseases, which coincide with inflammatory, allergic and/or proliferative processes:
   - Anal eczema
   - Fissures
   - Hemorrhoids
   - Idiopathic proctitis
(x) Eye diseases, which coincide with inflammatory, allergic and/or proliferative processes:
   - Allergic keratitis, uveitis, iritis
   - Conjunctivitis
   - Blepharitis
   - Optic neuritis
   - Chorioiditis
   - Sympathetic ophthalmia
(xi) Diseases of the ear-nose-throat area, which coincide with inflammatory, allergic and/or proliferative processes:
   - Allergic rhinitis, hay fever
   - Otitis externa, e.g., caused by contact dermatitis, infection, etc.
   - Otitis media
(xii) Neurological diseases, which coincide with inflammatory, allergic and/or proliferative processes:
   - Cerebral edema, primarily tumor-induced cerebral edema
   - Multiple sclerosis
   - Acute encephalomyelitis
   - Meningitis
   - Various forms of convulsions, e.g., infantile nodding spasms
   - Acute spinal cord injury
   - Stroke
(xiii) Blood diseases, which coincide with inflammatory, allergic and/or proliferative processes, such as, e.g.: M. Hodgkins or Non-Hodgkins lymphomas, thrombocythemias, erythrocytoses
   - Acquired hemolytic anemia
   - Idiopathic thrombocytopenia
(xiv) Tumor diseases, which coincide with inflammatory, allergic and/or proliferative processes, such as, e.g.: carcinomas or sarcomas
   - Acute lymphatic leukemia
   - Malignant lymphoma
   - Lymphogranulomatoses
   - Lymphosarcoma
   - Extensive metastases, mainly in breast, bronchial and prostate cancers
(xv) Endocrine diseases, which coincide with inflammatory, allergic and/or proliferative processes, such as, e.g.:
   - Endocrine orbitopathy
   - Thyreotoxic crisis
   - De Quervain's thyroiditis
   - Hashimoto's thyroiditis
   - Basedow's disease
   - Granulomatous thyroiditis
   - Lymphadenoid goiter
(xvi) Organ and tissue transplants, graft-versus-host disease
(xvii) Severe shock conditions, e.g., anaphylactic shock, systemic inflammatory response syndrome (SIRS)
(xviii) Substitution therapy in:
   - Innate primary suprarenal insufficiency, e.g., congenital adrenogenital syndrome
   - Acquired primary suprarenal insufficiency, e.g., Addison's disease, autoimmune adrenalitis, meta-infective tumors, metastases, etc.
   - Innate secondary suprarenal insufficiency, e.g., congenital hypopituitarism
   - Acquired secondary suprarenal insufficiency, e.g., meta-infective tumors, etc.
(xix) Emesis, which coincide with inflammatory, allergic and/or proliferative processes:
   - e.g., in combination with a 5-HT3 antagonist in cytostatic-agent-induced vomiting
(xx) Pains of inflammatory origins, e.g., lumbago
(xxi) Other different stages of disease including diabetes type I (insulin-dependent diabetes), osteoarthritis, Guillain-Barré syndrome, restenoses after percutaneous transluminal angioplasty, Alzheimer's disease, acute and chronic pain, arteriosclerosis, reperfusion injury, congestive heart failure, myocardial infarction, thermal injury, multiple organ injury secondary to trauma, acute purulent meningitis, necrotizing enterocolitis and syndromes associated with hemodialysis, leukopheresis, and granulocyte transfusion.

Moreover, the compounds of general formula I according to the invention can be used for treatment and prophylaxis of additional pathologic conditions that are not mentioned above, for which synthetic glucocorticoids are now used (see in this respect Hatz, H. J., Glucocorticoide: Immunologische Grundlagen, Pharmakologie und Therapierichtlinien, Wissenschaftliche Verlagsgesellschaft mbH, Stuttgart, 1998).

All previously mentioned indications (i) to (xx) are described in more detail in Hatz, H. J., Glucocorticoide: Immunologische Grundlagen, Pharmakologie und Therapierichtlinien, Wissenschaftliche Verlagsgesellschaft mbH, Stuttgart, 1998.

For the therapeutic actions in the above-mentioned pathologic conditions, the suitable dose varies and depends on, for example, the active strength of the compound of general formula I, the host, the type of administration, and the type and severity of the conditions that are to be treated, as well as the use as a prophylactic agent or therapeutic agent.

In addition, the invention provides:
(i) The use of one of the compounds of formula I according to the invention or mixture thereof for the production of a medication for treating a DISEASE;
(ii) A process for treating a DISEASE, said process comprises an administration of an amount of the compound according to the invention, wherein the amount suppresses the disease and wherein the amount of compound is given to a patient who requires such a medication;
(iii) A pharmaceutical composition for treating a DISEASE, said treatment comprises one of the compounds according to the invention or mixture thereof and at least one pharmaceutical adjuvant and/or vehicle.

In general, satisfactory results can be expected in animals when the daily doses comprise a range of 1 µg to 100,000 µg of the compound according to the invention per kg of body weight. In the case of larger mammals, for example the human, a recommended daily dose lies in the range of 1 µg to 100,000 µg per kg of body weight. Preferred is a dose of 10 to 30,000 µg per kg of body weight, and more preferred is a dose of 10 to 10,000 µg per kg of body weight. For example, this dose is suitably administered several times daily. For treating acute shock (e.g., anaphylactic shock), individual doses can be given that are significantly above the above-mentioned doses.

The formulation of the pharmaceutical preparations based on the new compounds is carried out in a way that is known in the art by the active ingredient being processed with the vehicles that are commonly used in galenicals, fillers, substances that influence decomposition, binding agents, moisturizers, lubricants, absorbents, diluents, flavoring correctives, coloring agents, etc., and converted into the desired form of administration. In this case, reference is made to Remington's Pharmaceutical Science, 15th Edition, Mack Publishing Company, East Pennsylvania (1980).

For oral administration, especially tablets, coated tablets, capsules, pills, powders, granulates, lozenges, suspensions, emulsions or solutions are suitable.

For parenteral administration, injection and infusion preparations are possible.

For intra-articular injection, correspondingly prepared crystal suspensions can be used.

For intramuscular injection, aqueous and oily injection solutions or suspensions and corresponding depot preparations can be used.

For rectal administration, the new compounds can be used in the form of suppositories, capsules, solutions (e.g., in the form of enemas) and ointments both for systemic and for local treatment.

For pulmonary administration of the new compounds, the latter can be used in the form of aerosols and inhalants.

For local application to eyes, outer ear channels, middle ears, nasal cavities, and paranasal sinuses, the new compounds can be used as drops, ointments and tinctures in corresponding pharmaceutical preparations.

For topical application, formulations in gels, ointments, fatty ointments, creams, pastes, powders, milk and tinctures are possible. The dosage of the compounds of general formula I should be 0.01%-20% in these preparations to achieve a sufficient pharmacological action.

The invention also comprises the compounds of general formula I according to the invention as therapeutic active ingredients.

In addition, the compounds of general formula I according to the invention are part of the invention as therapeutic active ingredients together with pharmaceutically compatible and acceptable adjuvants and vehicles.

The invention also comprises a pharmaceutical composition that contains one of the pharmaceutically active compounds according to the invention or mixtures thereof or a pharmaceutically compatible salt thereof and a pharmaceutically compatible salt or pharmaceutically compatible adjuvants and vehicles.

The compounds of general formula (I) according to the invention can optionally also be formulated and/or administered in combination with other active ingredients.

The invention therefore also relates to combination therapies or combined compositions, wherein a compound of general formula (I) or a pharmaceutically acceptable salt thereof, or a pharmaceutical composition that contains a compound of general formula (I) or a pharmaceutically acceptable salt thereof, is administered either simultaneously (optionally in the same composition) or in succession together with one or more pharmaceutical agents for treating one of the above-mentioned pathologic conditions. For example, for treatment of rheumatoid arthritis, osteoarthritis, COPD (chronic obstructive lung disease), asthma or allergic rhinitis, a compound of general formula (I) of this invention can be combined with one or more pharmaceutical agents for treating such a condition. When such a combination is administered by inhalation, the pharmaceutical agent that is to be combined can be selected from the following list:
- A PDE4 inhibitor including an inhibitor of the PDE4D isoform,
- A selective β.sub2.adrenoceptor agonist, such as, for example, metaproterenol, isoproterenol, isoprenaline, albuterol, salbutamol, formoterol, salmeterol, terbutaline, orcipresnaline, bitolterol mesylate, pirbuterol or indacaterol;
- A muscarine receptor antagonist (for example, an M1, M2 or M3 antagonist, such as, for example, a more selective M3 antagonist), such as, for example, ipratropium bromide, tiotropium bromide, oxitropium bromide, pirenzepine or telenzepine;
- A modulator of the chemokine receptor function (such as, for example, a CCR1 receptor antagonist); or
- An inhibitor of the p38 kinase function.

For another subject of this invention, such a combination with a compound of general formula (I) or a pharmaceutically acceptable salt thereof is used for treatment of COPD, asthma or allergic rhinitis and can be administered by inhalation or orally in combination with xanthine (such as, for example, aminophylline or thyeophylline), which also can be administered by inhalation or orally.

### Experimental Part:

| **Abbreviation** | **meaning** |
|---|---|
| Ac | acetyl |
| Boc | tert-butyloxycarbonyl |
| br | broad |
| Cl | chemical ionisation |
| d | doublet |
| dd | doublet of doublet |
| DCM | dichloromethane |
| DIPEA | N,N-diisopropylethyl amine |
| DMF | N,N-dimethylformamide |
| DMSO | dimethyl sulfoxide |
| eq. | equivalent |
| ESI | electrospray ionisation |
| EtOAc | ethylacetate |
| GP | general procedure |
| HPLC | high performance liquid chromatography |
| LC-MS | liquid chromatography mass spectrometry |
| m | Multiplet |
| mc | centred multiplet |
| MS | mass spectrometry |
| NMR | nuclear magnetic resonance spectroscopy : chemical shifts (δ) are given in ppm. |
| Pg | protecting group |
| q | quartet |
| rf | at reflux |
| r.t. or rt | room temperature |
| s | singlet |
| sept. | septet |
| t | triplet |
| TEA | triethylamine |
| TLC | thin layer chromatography |
| TFA | trifluoroacetic acid |
| THF | Tetrahydrofuran |
| br | broad |
| Cl | chemical ionisation |
| d | doublet |
| dd | doublet of doublet |
| DCM | dichloromethane |
| DIPEA | N,N-diisopropylethyl amine |
| DMF | N,N-dimethylformamide |
| DMSO | dimethyl sulfoxide |
| eq. | equivalent |
| ESI | electrospray ionisation |
| GP | general procedure |
| HCL | hydrochloric acid |
| HPLC | high performance liquid chromatography |
| LC-MS | liquid chromatography mass spectrometry |
| m | Multiplet |
| mc | centred multiplet |
| MS | mass spectrometry |
| NMR | nuclear magnetic resonance spectroscopy : chemical shifts (δ) are given in ppm. |
| Pg | protecting group |
| q | quartet |
| rf | at reflux |
| r.t. or rt | room temperature |
| s | singlet |
| sept. | septet |
| t | triplet |
| TEA | triethylamine |
| TLC | thin layer chromatography |
| TFA | trifluoroacetic acid |
| THF | Tetrahydrofuran |

The various aspects of the invention described in this application are illustrated by the following examples which are not meant to limit the invention in any way.

### Example 1

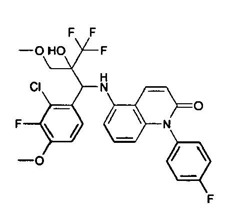

### 5-{[1-(2-Chloro-3-fluoro-4-methoxyphenyl)-3,3,3-trifluoro-2-hydroxy-2-([methoxymethyl)propyl]amino}-1H-quinolin-2-one

### 2-Chloro-3-fluoro-4-methoxybenzaldehyde

1g (6.2 mmol) 3-Chloro-2-fluoroanisole in 20 ml THF was cooled to -70°C and 2.7 ml of a 2.5 M n-butyl lithium solution in hexane were added. After one hour at -70° 3.93 ml DMF in 7 ml THF were added at -70°C and the mixture is stirred another hour at -70°C. 15 ml of a 1 M aqueous hydrochloric acid were added and the reaction was warmed to ambient temperature over 18 hours. The reaction mixture was partitioned between diethyl ether and water. The aqueous phase was extracted with diethyl ether, the combined organic phases were washed with brine, dried over sodium sulfate and evaporated. The crude product was purified by chromatography on silica gel to yield 0.25 g 2-chloro-3-fluoro-4-methoxybenzaldehyde. ¹H-NMR (CDCl₃); δ = 3.98 (s, 3H), 6.98 (dd, 1H), 7.75 (dd, 1H), 10.30 (s, 1H).

### 5-Amino-7-fluoro-1H-quinolin-2-one

To a solution of 2-bromo-3-fluoroaniline (6.5 g, 34.17 mmol) and pyridine (2.7 g, 34.17 mmol) in 20 ml of dichloromethane, cinnamoyl chloride (5.95 g, 35.88 mol) in 10 ml dichloromethane were added dropwise and mixture was refluxed for 30 min. The reaction mixture was diluted with dichloromethane, the organic layer washed with diluted hydrochloric acid , saturated sodium carbonate solution, water, and dried (sodium sulfate). The solvent was removed *in vacuo* to give 10.5 g of N-(2-bromo-3-fluorophenyl)-3-phenylacrylamide. To a solution of N-(2-bromo-3-fluorophenyl)-3-phenylacrylamide (10.5 g, 32.8 mmol) in 70 ml of chlorobenzene at 130 °C aluminum trichloride (21.9 g, 0.164 mol) was added portionwise, the mixture was stirred at this temperature 2 h and poured in ice-water. The precipitate was filtered off and dried. Yield 6.05 g (76 %). 6 g (24.8 mmol) of 8-bromo-7-fluoro-1 H-quinolin-2-one were refluxed in 30 mL of phosphorus oxychloride during 2 h, then poured on ice, extracted with benzene. the benzene extract dried (sodium sulfate) to yield yield 6.1 g 8-bromo-2-chloro-7-fluoroquinoline after solvent removal. To a mixture of 10 ml 10%-oleum and 1.4 g (22.2 mmol) of fuming nitric acid 8-bromo-2-chloro-7-fluoroquinoline (4.8 g 18.5 mmol) was added portionwise. The mixture was heated at 100 °C for 2 h. Additional nitric acid (0.17 g) was added and stirred for additional 1 h. The reaction mixture was poured in ice-water, extracted with ethyl acetate, filtered through silica gel, and crystallized from heptane-toluene to yield 2.3 g (50 %) 8-bromo-2-chloro-7-fluoro-5-nitroquinoline. 2.3 g (7.54 mmol) of 8-bromo-2-chloro-7-fluoro-5-nitroquinoline were heated at 100 °C for 5 h in a solution containing 16 ml of acetic acid, 3.2 ml of water and 5 ml of conc. HCl. The mixture was poured in water, the formed precipitate was filtered off, stirred in EtOAc and filtered to yield 1.71 g. 8-bromo-7-fluoro-5-nitro-1*H*-quinolin-2-one. To a suspension 1.7 g (5.92 mmol) of 8-bromo-7-fluoro-5-nitro-1*H*-quinolin-2-one and 2.3 g (35.5 mmol) of ammonium formate in 10 ml of ethanol 0.1 g 10 % palladium-carbon were added, and stirred for 2h at 60 °C. A solid disappeared and then formed again. The precipitate was filtered off, dissolved in 3 ml of DMSO and filtered through silica gel. 15 ml of water were added to the eluate, the precipitate was filtered off and dried to yield 0.5 g (47 %) 5-amino-7-fluoro-1 H-quinolin-2-one. ¹H-NMR (DMSO-d₆); δ = 6.14 (dd, 1H), 6.20 (dd, 1H), 6.23 (d, 1H), 6.27 (br, 2H), 8.06 (d, 1H), 11.50 (br., 1H).

### 5-{[(2-Chloro-3-fluoro-4-methoxyphenyl)(2-trifluoromethyl-oxiranyl)methyl]amino}-7-fluoro-1H-quinolin-2-one

To 1.6 g (9 mmol) 5-amino-7-fluoro-1*H*-quinolin-2-one and 1.69 g (9 mmol) 2-chloro-3-fluoro-4-methoxybenzaldehyde in 27 ml toluene and 8 ml 1,4-dioxane were added 1.96 ml acetic acid and 7 ml tetrabutyl orthotitanate. The mixture was heated over 20 hours to 110°C, cooled to room temperature and poured into aqueous ammonium fluoride solution. Ethyl acetate was added and the mixture was stirred vigorously for 1 hour. Phases were separated and addition of ethyl acetate was repeated two times while stirring was done under reflux and phases were separated while they were still hot. The combined organic phases were concentrated and the residue was purified by flash chromatography on silica gel (ethyl acetate, then methanol in dichloromethane 15% to 20%) to yield 2.17 g of 5-{[1-(2chloro-3-fluoro-4-methoxyphenyl)methylidene]-amino}-7-fluoro-1*H*-quinolin-2-one. 465 mg sodium hydride (55% in mineraloil, 9.7 mmol) were washed with dry THF and suspended together with 2.6 g (7.5 mmol) of 5-{[1-(2-chloro-3-fluoro-4-methoxyphenyl)methylidene]amino}-7- fluoro-1*H*-quinolin-2-one in 90 ml THF. *t-*Butyldimethylsilyl chloride was added as solid and the mixture was stirred for 3.5 hours while it becomes a clear solution. In parallel 0.96 ml 1,1,1-trifluoro-2,3-epoxypropane in 24 ml THF and 7 ml hexane were cooled to -100°C and 4.5 ml of a 2.5 M n-butyl lithium solution in hexane were added over 10 minutes while the temperature did not exceeded -95°C. 10 Minutes after complete addition the previously prepared 1-{*t*-butyldimethylsilyl}-5-{[1-(2-chloro-3-fluoro-4-methoxyphenyl)methylidene]amino}-7-fluoroquinolin-2-one solution in THF was added over 30 minutes while the temperature did not exceeded -95°C. After 3 hours at -100°C 7.5 ml diethyl ether was added and the reaction mixture was warmed to room temperature over one hour. The reaction was quenched by addition of saturated ammonium chloride solution. After stirring for 30 minutes the phases were separated and the aqueous layer was extracted with dichloromethan, the combined organic phases were washed with brine, dried over sodium sulphate and then evaporated. Flash chromatography on silica gel (ethyl acetate in hexane 50 to 100%) yielded 2.14 g of 5-{[(2-chloro-3-fluoro-4-methoxyphenyl)(2-trifluoromethyloxiranyl)methyl]amino}-7-fluoro-1*H*-quinolin-2-one.
¹H-NMR (DMSO-d₆); δ = 2.62 (m, 1H), 3.29 (d, 1H), 3.87 (s, 3H), 5.49 (d, 1H), 5.83 (d, 1H), 6.34 (d, 1H), 6.37 (d, 1H), 7.04 (d, 1H), 7.22 (dd, 1H), 7.44 (d, 1H), 8.31 (d, 1H), 11.63 (s, 1H).

### 5-{[1-(2-Chloro-3-fluoro-4-methoxyphenyl)-3,3,3-trifluoro-2-hydroxy-2-(methoxymethyl)propyl]amino}-7-fluoro-1H-quinolin-2-one

2.14 g (4.64 mmol) 5-{[(2-chloro-3-fluoro-4-methoxyphenyl)(2-trifluoromethyl-oxiranyl)methyl]amino}-7-fluoro-1*H*-quinolin-2-on was stirred with 2.57 g (7.9 mmol) caesium carbonate in 37 ml methanol. After 3 days water was added and the aqueous phase was extracted with ethyl acetate. The combined organic phases were washed with brine and dried over sodium sulphate. After removal of the solvent flash chromatography on silica gel (methanol in dichloromethane 0 to 5 %) yielded 0.98 g 5-{[1-(2-Chloro-3-fluoro-4-methoxyphenyl)-3,3,3-trifluoro-2-hydroxy-2-(methoxymethyl)propyl]amino}-7-fluoro-*1H*-quinolin-2-one.
¹H-NMR (CD₃OD); δ = 3.07 (d, 1H), 3.23 (s, 3H), 3.50 (d, 1H), 3.84 (s, 3H), 5.33 (s, 1H), 6.02 (dd, 1H), 6.29 (dd, 1H), 6.43 (d, 1H), 7.05 (dd, 1H), 7.47 (dd, 1H), 8.04 (d, 1 H).
25 mg (0.051 mmol) 5-{[1-(2-Chloro-3-fluoro-4-methoxyphenyl)-3,3,3-trifluoro-2-hydroxy-2-(methoxymethyl)propyl]amino}-7-fluoro-*1H*-quinolin-2-one, 14.2 mg (0.1 mmol) 4-fluorophenyl boronic acid and 10.1 mg (0.051 mmol) water free copper II acetate were stirred in 2.2 ml dichloromethane and 82 µl pyridine for 65 hours at room temperature. After removal of the solvent preparative thin layer chromatography on silica gel (ethyl acetate / hexane 1 :1) yielded 21 mg of the title compound.
¹H-NMR (CDCl₃); δ = 3.29 (d, 1H), 3.31 (s, 3H), 3.62 (d, 1H), 3.90 (s, 3H), 5.26 (d, 1H), 5.62 (dd, 1H), 5.98 (dd, 1H), 6.15 (d, 1H), 6.68 (d, 1H), 6.90 (dd, 1H), 7.22 (m, 3H),7.28 (dd, 2H), 7.85 (d, 1H).

### Example 2

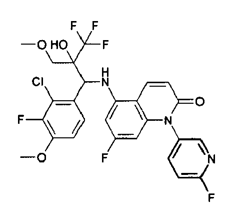

### 5-{[1-(2-Chloro-3-fluoro-4-methoxyphenyl)-3,3,3-trifluoro-2-hydroxy-2-(methoxymethyl)propyl]amino}-7-fluoro-1-(6-fluoropyridin-3-yl)-1H-quinolin-2-one

25 mg (0.051 mmol) 5-{[1-(2-Chloro-3-fluoro-4-methoxyphenyl)-3,3,3-trifluoro-2-hydroxy-2-(methoxymethyl)propyl]amino}-7-fluoro-*1H*-quinolin-2-one, 14.3 mg (0.1 mmol) 6-fluoropyridin-3-yl boronic acid and 10.1 mg (0.051 mmol) water free copper acetate were stirred in 2.2 ml dichloromethane and 82 µl pyridine for 60 hours at room temperature. After removal of the solvent preparative thin layer chromatography on silica gel (ethyl acetate / hexane 1 :1) yielded 7.7 mg of the title compound.
¹H-NMR (CDCl₃); δ = 3.26 (d, 1H), 3.29 (s, 3H), 3.62 (d, 1H), 3.89 (s, 3H), 5.24 (d, 1H), 5.58 (dd, 1H), 5.90 (dd, 1H), 6.18 (d, 1H), 6.65 (d, 1H), 6.90 (dd, 1H), 7.15 (ddd, 1H), 7.29 (m, 1H), 7.68 (ddd, 1H), 7.88 (d, 1 H), 8.11 (dd, 1H).

### Example 3

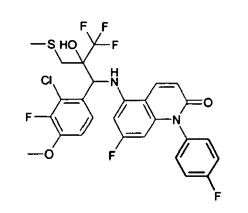

### 5-{[1-(2-Chloro-3-fluoro-4-methoxyphenyl)-3,3,3-trifluoro-2-hydroxy-2-([methylsulfanyl]methyl)-propyl]amino}-7-fluoro-1-(4-fluorophenyl)quinolin-2-one

### 5-{[1-(2-Chloro-3-fluoro-4-methoxyphenyl)-3,3,3-trifluoro-2-hydroxy-2-([methylsulfanyl]methyl)propyl]amino}-7-fluoro-1H-quinolin-2-one

To 65 mg (0.14 mmol) 5-{[(2-chloro-3-fluoro-4-methoxyphenyl)(2-trifluoromethyl-oxiranyl)methyl]amino}-7-fluoro-1H-quinolin-2-on and 92 mg (0.28 mmol) caesium carbonate in 0.5 ml DMF were added 0.21 ml of a 1 M solution of methyl mercaptan in DMF. The mixture was stirred vigorously for 20 hours and water was added. The aqueous layer was extracted with ethyl acetate, the organic phases washed with brine and dried over sodium sulphate. After removal of the solvent thin layer chromatography on silica gel (ethyl acetate) yielded 22 mg of 5-{[1-(2-Chloro-3-fluoro-4-methoxyphenyl)- 3,3,3-trifluoro-2-hydroxy2-([methylsulfanyl]methyl)propyl]amino}-7-fluoro-*1H*-quinolin-2-one.
¹H-NMR (CDCl₃); δ = 1.91 (s, 3H), 2.68 (d, 1 H), 3.04 (d, 1 H), 3.87 (s, 3H), 5.21 (d, 1 H), 5.80 (dd, 1 H), 5.94 (d, 1 H), 6.38 (dd, 1 H), 6.57 (d, 1 H), 6.87 (dd, 1 H), 7.24 (dd, 1H), 7.84 (d, 1 H).
Analogously to example 1 5-{[1-(2-Chloro-3-fluoro-4-methoxyphenyl)-2-([methylsulfanyl]methyl)-3,3,3-trifluoro-2-hydroxypropyl]amino}-7-fluoro-*1H-*quinolin-2-one can be reacted with 4-fluorophenyl boronic acid in the presence of water free copper acetate to yield the title compound.

### Example 4

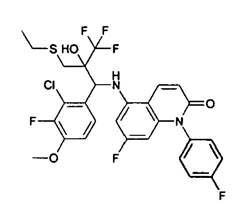

### 5-{[1-(2-Chloro-3-fluoro-4-methoxyphenyl)-2-([ethylsulfanyl]methyl)-3,3,3-trifluoro-2-hydroxypropyl]amino}-7-fluoro-1-(4-fluorophenyl)-1H-quinolin-2-one

### 5-{[1-(2-Chloro-3-fluoro-4-methoxyphenyl)-2-([ethylsulfanyl]ethyl)-3,3,3-trifluoro-2-hydroxypropyl]amino}-7-fluoro-1H-quinolin-2-one

To 66 mg (0.14 mmol) 5-{[(2-chloro-3-fluoro-4-methoxyphenyl)(2-trifluoromethyl-oxiranyl)methyl]amino}-7-fluoro-1*H*-quinolin-2-on and 93 mg (0.29 mmol) caesium carbonate in 0.6 ml DMF were added 16 µl (0.22 mmol) of ethyl mercaptan in DMF. The mixture was stirred vigorously for 20 hours and water was added. The aqueous layer was extracted with ethyl acetate, the organic phases washed with brine and dried over sodium sulphate. After removal of the solvent thin layer chromatography on silica gel (ethyl acetate) yielded 14 mg of 5-{[1-(2-chloro-3-fluoro-4-methoxyphenyl)-2-([ethylsulfanyl]ethyl)-3,3,3-trifluoro-2-hydroxypropyl]amino}-7-fluoro-*1H*-quinolin-2-one.
¹H-NMR (CDCl₃); δ = 1.07 (t, 3H), 2.27 (dq, 2H), 2.69 (d, 1 H), 3.06 (d, 1 H), 3.88 (s, 3H), 5.20 (d, 1 H), 5.79 (dd, 1 H), 5.92 (d, 1 H), 6.37 (dd, 1 H), 6.57 (d, 1 H), 6.87 (dd, 1 H), 7.24 (dd, 1 H), 7.84 (d, 1 H).
Analogously to example 1 5-{[1-(2-Chloro-3-fluoro-4-methoxyphenyl)-2-([methylsulfanyl]ethyl)-3,3,3-trifluoro-2-hydroxypropyl]amino}-7-fluoro-*1H-*quinolin-2-one can be reacted with 4-fluorophenyl boronic acid in the presence of water free copper acetate to yield the title compound.

### Example 5

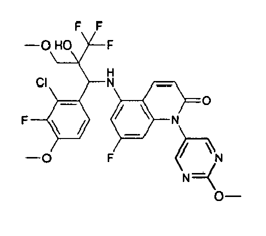

### 5-{[1-(2-Chloro-3-fluoro-4-methoxyphenyl)-2-(ethoxymethyl)-3,3,3-trifluoro-2-hydroxypropyl]amino}-7-fluoro-1-(2-methoxypyrimidine-5-yl)-1H-quinolin-2-one

100 mg (0.2 mmol) 5-{[1-(2-Chloro-3-fluoro-4-methoxyphenyl)-3,3,3-trifluoro-2-hydroxy-2-(methoxymethyl)propyl]amino}-7-fluoro-*1H*-quinolin-2-one, 62.5 mg (0.41 mmol) 2-methoxypyrimidine-5-yl boronic acid and 37 mg (0.2 mmol) water free copper acetate were stirred in 8.6 ml dichloromethane and 330 µl pyridine for 60 hours at room temperature. Then additional 35 mg 2-methoxypyrimidine-5-yl boronic acid and 20 mg water free copper acetate were added. After 4 days the solvent was removed column chromatography on silica gel (ethyl acetate / methanol 0 to 10%) and additional preparative thin layer chromatography on silica gel (hexane / acetone 1 :1) yielded 9.4 mg of the title compound.
¹H-NMR (CDCl₃); δ = 3.25 (d, 1H), 3.29 (s, 3H), 3.64 (d, 1H), 3.89 (s, 3H), 4.09 (s, 3H), 5.24 (d, 1H), 5.66 (dd, 1H), 5.90 (dd, 1H), 6.16 (d, 1H), 6.65 (d, 1H), 6.90 (dd, 1H), 7.27 (d, 1H), 7.86 (d, 1H), 8.37 (d, 1H), 8.44 (d, 1H).

### Example 6

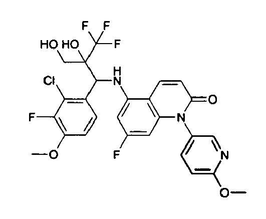

### 5-{[1-(2-Chloro-3-fluoro-4-methoxyphenyl)-3,3,3-trifluoro-2-hydroxy-2-(hydroxymethyl)propyl]amino}-7-fluoro-1-(6-methoxypyridine-3-yl)-1H-quinolin-2-one

### 5-{[1-(2-Chloro-3-fluoro-4-methoxyphenyl)-3,3,3-trifluoro-2-hydroxy-2-(hydroxymethyl)propyl]amino}-7-fluoro-1H-quinolin-2-one

250 mg (0.54 mmol) 5-{[(2-chloro-3-fluoro-4-methoxyphenyl)(2-trifluoromethyl-oxiranyl)methyl]amino}-7-fluoro-1*H*-quinolin-2-on were stirred with 353 mg (1.1 mmol) caesium carbonate in 3 ml DMF, 1.9 ml water and 0.5 ml DMSO. Water was added and the aqueous phase was extracted with ethyl acetate. The combined organic phases were washed with brine and dried over sodium sulphate. After removal of the solvent flash chromatography on silica gel (methanol in dichloromethan 0 to 5 %) yielded 0.98 g of 5-{[1-(2-Chloro-3-fluoro-4-methoxyphenyl)-3,3,3-trifluoro-2-hydroxy-2-(hydroxymethyl)propyl]amino}-7-fluoro-1H-quinolin-2-one.
¹H-NMR (CD₃OD); δ = 3.60 (d, 1 H), 3.71 (d, 1 H), 3.85 (s, 3H), 5.34 (s, 1 H), 5.96 (dd, 1 H), 6.29 (dd, 1 H), 6.45 (d, 1 H), 7.06 (dd, 1 H), 7.51 (dd, 1 H), 8.04 (d, 1 H). 20 mg (0.042 mmol) 5-{[1-(2-Chloro-3-fluoro-4-methoxyphenyl)-3,3,3-trifluoro-2-hydroxy-2-(hydroxymethyl)propyl]amino}-7-fluoro-1*H*-quinolin-2-one, 8.0 mg (0.052 mmol) 6-methoxypyridin-3-yl boronic acid and 8.3 mg (0.042 mmol) water free copper(II)acetate were stirred in 1.8 ml dichloromethane and 67 µl pyridine for 80 hours at room temperature. Preparative thin layer chromatography of the reaction mixture on silica gel (dichloromethane / methanol 9 :1) yielded 1.7 mg of the title compound.
¹H-NMR (CDCl₃); δ = 3.48 (d, 1H), 3.88 (s, 3H), 4.00 (s, 3H), 4.02 (d, 1H), 5.25 (br, 1H), 5.69 (dd, 1H), 5.87 (dd, 1H), 6.21 (br, 1H), 6.67 (d, 1H), 6.93 (m, 2H), 7.35 (dd, 1H), 7.42 (m, 1H), 7.88 (d, 1H), 7.98 (s, 1H).

### Example 7

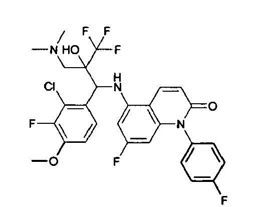

### 5-{[1-(5-Chloro-3-fluoro-2-methoxyphenyl)-2-(dimethylaminomethyl)-3,3,3-trifluoro2-hydroxypropyl]amino}-7-fluoro-(4-fluorophenyl)-1H-quinolin-2-one

### 5-{[1-(5-Chloro-3-fluoro-2-methoxyphenyl)-2-(dimethylaminomethyl)-3,3,3-trifluoro2-hydroxypropyl]amino}-7-fluoro-1H-quinolin-2-one

150 mg (0.33 mmol) 5-{[(5-chloro-3-fluoro-2-methoxyphenyl)(2-trifluoromethyl-oxiranyl)methyl]amino}-7-fluoro-1*H*-quinolin-2-on were stirred with 69 mg (0.65 mmol) lithium perchlorate and molecular sieve in 3.3 ml of a 2 M solution of dimethyl amine in THF and additional 5 ml THF for 20 hours at 60°C in a closed pressure vessel. After cooling to room temperature the solids were filtered off and washed with Ethyl acetate. After removal of the solvent chromatography on silica gel (hexane / ethyl acetate 50% - 100%) yielded 129 mg of 5-{[1-(2-Chloro-3-fluoro-4-methoxyphenyl)-2-(dimethylaminomethyl)-3,3,3-trifluoro-2-hydroxypropyl]amino}-7-fluoro-1*H*-quinolin-2-one.
¹H-NMR (CDCl₃); δ = 1.68 (br, 3H), 2.30 (d, 1H), 2.36 (br, 3H), 2.77 (d, 1H), 3.87 (s, 3H), 5.09 (d, 1H), 5.80 (d, 1H), 5.98 (d, 1H), 6.35 (d, 1H), 6.56 (d, 1H), 6.88 (dd, 1H), 7.32 (d, 1H), 7.85 (d, 1H), 11.53 (br., 1H).
25 mg (0.049 mmol) 5-{[1-(2-Chloro-3-fluoro-4-methoxyphenyl)-2-(dimethylaminomethyl)-3,3,3-trifluoro-2-hydroxypropyl]amino}-7-fluoro-*1H-*quinolin-2-one, 13.8 mg (0.1 mmol) 4-fluorophenyl boronic acid and 9.0 mg (0.049 mmol) water free copper(II)acetate were stirred in 2.1 ml dichloromethane and 80 µl pyridine for 48 hours at room temperature. Preparative thin layer chromatography of the reaction mixture on silica gel (ethyl acetate) yielded 5.1 mg of the title compound.
¹H-NMR (CDCl₃); δ = 2.17 (s, 6H), 2.30 (d, 1H), 2.79 (d, 1H), 3.88 (s, 3H), 5.09 (d, 1H), 5.57 (dd, 1H), 5.82 (dd, 1H), 6.05 (d, 1H), 6.67 (d, 1H), 6.90 (dd, 2H), 7.20 (m, 4H), 7.35 (dd, 1H), 7.87 (d, 1H).

The following compounds are made using the methods as indicated in the table below:

| **Example No.** | **Structure** | **Preparation method (Ref. Example No.)** |
|---|---|---|
| 8 | 5-{[1-(2-Chloro-3-fluoro-4-methoxyphenyl)-3,3,3-trifluoro-2-hydroxy-2-(hydroxymethyl)-propyl]amino}-7-fluoro-1-(6-fluoropyridine-3-yl)-1*H*-quinolin-2-one | 6 |
| | 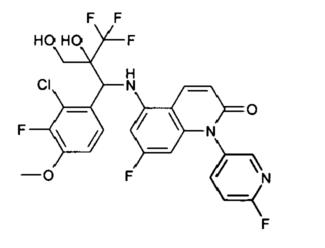 | |
| 9 | 5-{[1-(4-Chloro-3-fluoro-2-methoxyphenyl)-3,3,3-trifluoro-2-hydroxy-2-(methoxymethyl)propyl]amino}-1-(2-fluoropyrimidin-5-yl)-1*H*-quinolin-2-one | 1 |
| | 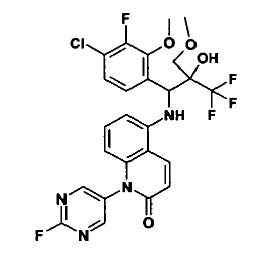 | |
| 10 | 5-{[2-(Ethoxymethyl)-1-(3-fluoro-4-methoxyphenyl)-3,3,3-trifluoro-2-hydroxy-propyl]amino}-1-(pyrimidin-4-yl)-quinolin-2-one-1H--quinolin-2-one | 1 |
| | 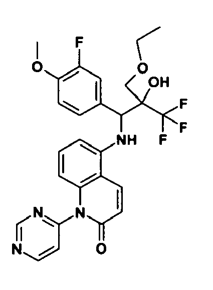 | |
| 11 | 1-(4-Fluorophenyl)-5-{[3,3,3-trifluoro-1-(3-fluoro-4-methoxy-2-methylphenyl)-2-hydroxy-2-({methylsulfonyl}methyl)propyl]amino}-1*H*-quinolin-2-one | 3 |
| | 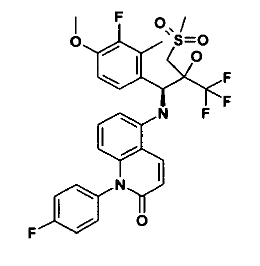 | |
| 12 | 5-{[1-(2-Chloro-3-fluoro-4-methoxyphenyl)-2-({ethylsulfonyl}methyl)-3,3,3-trifluoro-2-hydroxypropyl]amino}-1-(4-fluorophenyl)-7-methyl-1*H*-quinolin-2-one | 3 |
| | 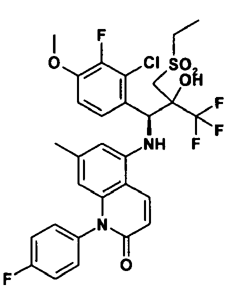 | |
| 13 | 5-{[1-(2-Chloro-4-ethylphenyl)-3,3,3-trifluoro-2-hydroxy-2-(methoxymethyl)propyl]amino}-1-(2-methoxypyrimidin-5-yl)-1*H*-quinolin-2-one | 1 |
| | 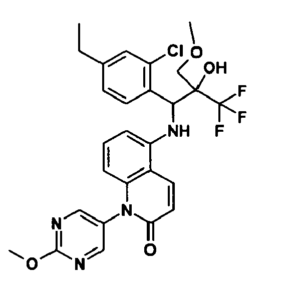 | |
| 14 | 5-{[1-(4-Chloro-3-fluoro-2-hydroxyphenyl)-3,3,3-trifluoro-2-hydroxy-2-(hydroxymethyl)propyl]amino}-7-fluoro-1-(6-fluoropyridin-3yl)-1*H*-quinolin-2-one | 6 |
| | 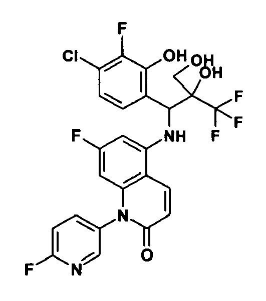 | |
| 15 | 5-{[1-(2-Chloro-3-fluoro-4-methoxyphenyl)-2-({dimethylamino}methyl)-3,3,3-trifluoro-2-hydroxypropyl]amino}-7-fluoro-1-(6-fluoropyridin-2-yl)-1*H*-quinolin-2-one | 7 |
| | 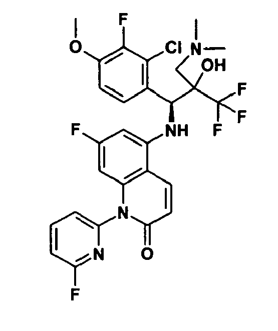 | |
| 16 | 5-{[2-({Ethylsulfanyl}methyl)-1-(3-fluoro-4-methoxyphenyl)-3,3,3-trifluoro-2-hydroxy-propyl]amino}-1-(2-methylpyrimidin-5-yl)-1*H*-quinolin-2-one | 3 |
| | 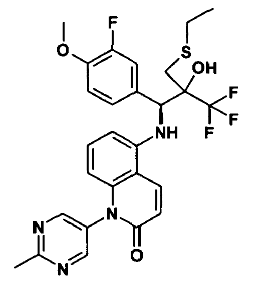 | |
| 17 | 5-{[1-(2-Chloro-4-methylphenyl)-3,3,3-trifluoro-2-hydroxy-2-(methoxymethyl)propyl]amino}-1-(2-methylpyridin-4-yl)-1*H*-quinolin-2-one | 1 |
| | 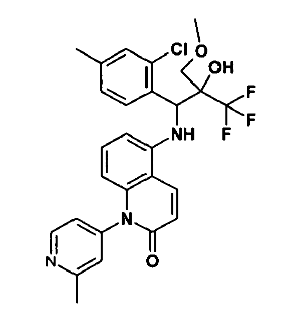 | |
| 18 | 7-Fluoro-1-(6-fluoropyridin-3-yl)-5-{[3,3,3-trifluoro-1-(3-fluoro-4-methylphenyl)-2-hydroxy-2-(hydroxymethyl)propyl]amino}-1*H*-quinolin-2-one | 6 |
| | 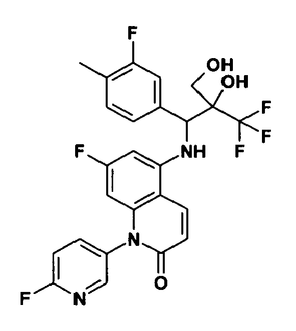 | |

## Claims

1. Compounds of general formula I 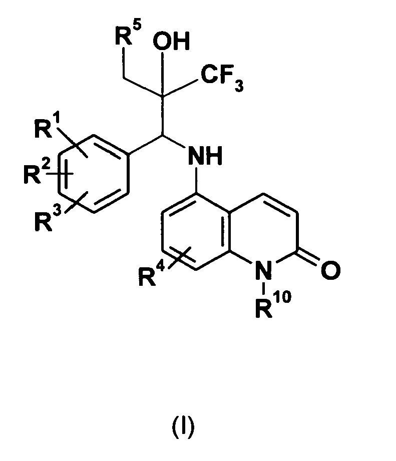 In which
R¹and R² independently of one another, mean a hydrogen atom, a hydroxy group, a halogen atom, an optionally substituted (C₁-C₁₀)-alkyl group, an optionally substituted (C₁-C₁₀)-alkoxy group, a (C₁-C₁₀)- alkylthio group, a (C₁-C₅)-perfluoroalkyl group, a cyano group, a nitro group,
or R¹ and R²
together mean a group that is selected from the groups -O-(CH₂)ₚ- O-, -O-(CH₂)ₚ-CH₂-, -O-CH=CH-, -(CH₂)ₚ₊₂-, -NH-(CH₂)ₚ₊₁, -N(C₁- C₃-alkyl)-(CH₂)ₚ₊₁, and -NH-N=CH-,
whereby p = 1 or 2, and the terminal oxygen atoms and/or carbon atoms and/or nitrogen atoms are linked to directly adjacent ring-carbon atoms,
or NR⁶R⁷,
whereby R⁶ and R⁷, independently of one another, mean hydrogen, C₁-C₅-alkyl or (CO)-(C₁-C₅)-alkyl,
R³ means a hydrogen atom, a hydroxy group, a halogen atom, a cyano group, an optionally substituted (C₁-C₁₀)-alkyl group, a (C₁- C₁₀)-alkoxy group, a (C₁-C₁₀)-alkylthio group, or a (C₁-C₅)- perfluoroalkyl group,
R⁴ means a hydrogen, halogen, hydroxy, (C₁-C₅)-alkyl, (C₁-C₅)alkoxy, (C₁-C₅)-alkylthio, (C₁-C₅)-perfluoroalkyl, cyano, nitro, NR⁷R⁸, COOR⁹, (CO)NR⁷R⁸ or a (C₁-C₅-alkylene)-O-(CO)-(C₁-C₅)alkyl group
R⁵ means a group selected from -(C₁-C₁₀)alkyl, which may be optionally partially or completely halogenated, -(C₁-C₁₀)alkenyl -(C₁-C₁₀)alkynyl, -R⁸, R⁸-(C₁-C₈)alkyl, R⁸-(C₂-C₈)alkenyl, R⁸-(C₂-C₈)alkynyl, -S-(C₁-C₁₀)-alkyl, -SO₂-(C₁-C₁₀)-alkyl -S-R⁸, -SO₂-R⁸, -CN -Hal, -O-(C₁-C₁₀)-alkyl, -NR⁶R⁷ wherein R⁶, R⁷ have the meaning defined above -O-R⁸, -OH
R⁸ means an aryl group which may optionally be substituted by 1-3 hydroxy, halogen, C₁-C₅-alkyl, C₁-C₅-alkoxy, cyano, CF₃, nitro, COO(C₁-C₅-alkyl) or C(O)OCH₂-phenyl or a heteroaryl group whereby the heteroaryl group may contain 1-3 hetero atoms which may optionally be substituted by 1-3 alkyl groups, hydroxy, halogen, cyano or C₁-C₅-alkoxy groups,
R⁹ means an hydrogen or a C₁-C₅-alkyl group
R¹⁰ is a group 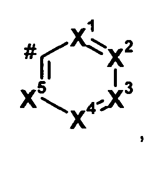 in which # denotes the point of attachment of the R¹⁰ group via a single bond,
X¹, X², X³, X⁴, X⁵ independently of one another are nitrogen, or a group C- R¹¹
in which R¹¹ is selected from the group consisting of hydrogen, halogen, cyano, nitro, hydroxy, (C₁-C₅)-alkoxy, (C₁-C₅)-alkyl, (C₁- C₅)-halo-alkyl, (C₁-C₅)halo-alkoxy and COOR⁹,
and the R¹⁰ group contains a maximum of 3 nitrogen atoms in the ring,
and their salts, solvates or salts of solvates.

2. Compounds of general formula I according to claim 1, in which
R¹and R² independently of one another, mean a hydrogen atom, a hydroxy group, a halogen atom, an optionally substituted (C₁-C₁₀)-alkyl group, an optionally substituted (C₁-C₁₀)-alkoxy group, a (C₁-C₁₀)- alkylthio group, a (C₁-C₅)-perfluoroalkyl group, a cyano group, a nitro group,
or R¹ and R²
together mean a group that is selected from the groups -O-(CH₂)ₚ- O-, -O-(CH₂)ₚ-CH₂-, -O-CH=CH-, -(CH₂)ₚ₊₂-, -NH-(CH₂)ₚ₊₁, -N(C₁- C₃-alkyl)-(CH₂)ₚ₊₁, and -NH-N=CH-,
whereby p = 1 or 2, and the terminal oxygen atoms and/or carbon atoms and/or nitrogen atoms are linked to directly adjacent ring-carbon atoms,
or NR⁶R⁷,
whereby R⁶ and R⁷, independently of one another, mean hydrogen, C₁-C₅-alkyl or (CO)-(C₁-C₅)-alkyl,
R³ means a hydrogen atom, a hydroxy group, a halogen atom, a cyano group, an optionally substituted (C₁-C₁₀)-alkyl group, a (C₁- C₁₀)-alkoxy group, a (C₁-C₁₀)-alkylthio group, or a (C₁-C₅)- perfluoroalkyl group,
R⁴ means a hydrogen, halogen, hydroxy, (C₁-C₅)-alkyl, (C₁-C₅)alkoxy,
R⁵ means a group selected from -(C₁-C₁₀)alkyl, which may be optionally partially or completely halogenated
-(C₁-C₁₀)alkenyl
-(C₁-C₁₀)alkynyl,
-S-(C₁-C₁₀)-alkyl,
-SO₂-(C₁-C₁₀)-alkyl
-CN
-Hal,
-O-(C₁-C₁₀)-alkyl,
-NR⁶R⁷ wherein R⁶, R⁷ have the meaning defined above
-OH
R¹⁰ is a group 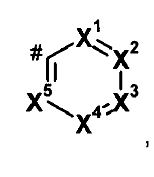 in which # denotes the point of attachment of the R¹⁰ group via a single bond,
X¹, X², X³, X⁴, X⁵ independently of one another are nitrogen, or a group C- R¹¹
in which R¹¹ is selected from the group consisting of hydrogen, halogen, cyano, hydroxy, (C₁-C₅)-alkoxy, (C₁-C₅)-alkyl, (C₁-C₅)- halo-alkyl,
and the R¹⁰ group contains a maximum of 2 nitrogen atoms in the ring,
, and their salts, solvates or salts of solvates.

3. Compounds of general formula I according to claim 1 to 2, in which
R¹ and R² independently of one another, mean a hydrogen atom, a hydroxy
group, a halogen atom, an optionally substituted (C₁-C₁₀)- alkyl group, an optionally substituted (C₁-C₁₀)-alkoxy group, a (C₁-C₁₀)-alkylthio group, a (C₁-C₅)-perfluoroalkyl group, a cyano group, a nitro group,
or NR⁶R⁷,
whereby R⁶ and R⁷, independently of one another, mean hydrogen, C₁-C₅-alkyl or (CO)-(C₁-C₅)-alkyl,
R³ means a hydrogen atom, a hydroxy group, a halogen atom, a cyano group, an optionally substituted (C₁-C₁₀)-alkyl group, a (C₁- C₁₀)-alkoxy group,
R⁴ means a hydrogen, fluoro, (C₁-C₅)-alkyl,
R⁵ means a group selected from
-(C₁-C₁₀)alkyl, which may be optionally partially or completely halogenated
-S-(C₁-C₁₀)-alkyl,
-SO₂-(C₁-C₁₀)-alkyl
-CN
-Hal,
-O-(C₁-C₁₀)-alkyl, -NR⁶R⁷ wherein R⁶, R⁷ have the meaning defined above -OH
R¹⁰ is a group 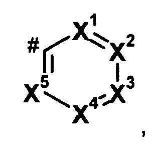 in which # denotes the point of attachment of the R¹⁰ group via a single bond,
X¹, X², X³, X⁴, X⁵ independently of one another are nitrogen, or a group C- R¹¹ in which R¹¹ is selected from the group consisting of hydrogen, halogen, (C₁-C₅)-alkoxy, (C₁-C₅)-alkyl,
and the R¹⁰ group contains a maximum of 2 nitrogen atoms in the ring, and their salts, solvates or salts of solvates.

4. Compounds according to claim 1 to 3 selected from the list consisting of:
5-{[1-(2-Chloro-3-fluoro-4-methoxyphenyl)-3,3,3-trifluoro-2-hydroxy-2-(methoxymethyl)propyl]amino}-7-fluoro-1-(4-fluorophenyl)quinolin-2-one
5-{[1-(2-Chloro-3-fluoro-4-methoxyphenyl)-3,3,3-trifluoro-2-hydroxy-2-(methoxymethyl)propyl]amino}-7-fluoro-1-(6-fluoropyridin-3yl)quinolin-2-one
5-{[1-(2-Chloro-3-fluoro-4-methoxyphenyl)-3,3,3-trifluoro-2-hydroxy-2-({methylsulfanyl}methyl)propyl]amino}-7-fluoro-1-(4-fluorophenyl)quinolin-2-one
5-{[1-(2-Chloro-3-fluoro-4-methoxyphenyl)-3,3,3-trifluoro-2-hydroxy-2-({ethylsulfanyl}methyl)propyl]amino}-7-fluoro-1-(6-fluoropyridin-3y)quinolin-2-one
5-{[1-(2-Chloro-3-fluoro-4-methoxyphenyl)-3,3,3-trifluoro-2-hydroxy-2-(methoxymethyl)propyl]amino}-7-fluoro-1-(2-methoxypyrimidin-5yl)quinolin-2-one
5-{[1-(2-Chloro-3-fluoro-4-methoxyphenyl)-3,3,3-trifluoro-2-hydroxy-2-({ethylsulfanyl}methyl)propyl]amino}-7-fluoro-1-(6-metoxypyridin-3yl)quinolin-2-one
5-{[1-(2-Chloro-3-fluoro-4-methoxyphenyl)-2-({dimethylamino}methyl)-3,3,3-trifluoro-2-hydroxypropyl]amino}-7-fluoro-1-(4-fluorophenyl)quinolin-2-one
5-{[1-(2-Chloro-3-fluoro-4-methoxyphenyl)-3,3,3-trifluoro-2-hydroxy-2-(hydroxymethyl)propyl]amino}-7-fluoro-1-(6-fluoropyridin-3yl)quinolin-2-one
5-{[1-(4-Chloro-3-fluoro-2-methoxyphenyl)-3,3,3-trifluoro-2-hydroxy-2-(methoxymethyl)propyl]amino}-1-(2-fluoropyrimidin-5-yl)-1*H*-quinolin-2-one
5-{[2-(Ethoxymethyl)-1-(3-fluoro-4-methoxyphenyl)-3,3,3-trifluoro-2-hydroxy-propyl]amino}-1-(pyrimidin-4-yl)-quinolin-2-one-1H--quinolin-2-one
1-(4-Fluorophenyl)-5-{[3,3,3-trifluoro-1-(3-fluoro-4-methoxy-2-methylphenyl)-2-hydroxy-2-({methylsulfonyl}methyl)propyl]amino}-1*H-*quinolin-2-one
5-{[1-(2-Chloro-3-fluoro-4-methoxyphenyl)-2-({ethylsulfonyl}methyl)-3,3,3-trifluoro-2-hydroxypropyl]amino}-1-(4-fluorophenyl)-7-methyl-1*H*-quinolin-2-one
5-{[1-(2-Chloro-4-ethylphenyl)-3,3,3-trifluoro-2-hydroxy-2-(methoxymethyl)propyl]amino}-1-(2-methoxypyrimidin-5-yl)-1*H*-quinolin-2-one
5-{[1-(4-Chloro-3-fluoro-2-hydroxyphenyl)-3,3,3-trifluoro-2-hydroxy-2-(hydroxymethyl)propyl]amino}-7-fluoro-1-(6-fluoropyridin-3yl)-1*H*-quinolin-2-one
5-{[1-(2-Chloro-3-fluoro-4-methoxyphenyl)-2-({dimethylamino}methyl)-3,3,3-trifluoro-2-hydroxypropyl]amino}-7-fluoro-1-(6-fluoropyridin-2-yl)-1*H-*quinolin-2-one
5-{[2-({Ethylsulfanyl}methyl)-1-(3-fluoro-4-methoxyphenyl)-3,3,3-trifluoro-2-hydroxy-propyl]amino}-1-(2-methylpyrimidin-5-yl)-1*H*-quinolin-2-one
5-{[1-(2-Chloro-4-methylphenyl)-3,3,3-trifluoro-2-hydroxy-2-(methoxymethyl)propyl]amino}-1-(2-methylpyridin-4-yl)-1*H*-quinolin-2-one
7-Fluoro-1-(6-fluoropyridin-3-yl)-5-{[3,3,3-trifluoro-1-(3-fluoro-4-methylphenyl)-2-hydroxy-2-(hydroxymethyl)propyl]amino}-1*H*-quinolin-2-one

5. Use of the compounds according to formula I of at least one of claims 1-4 for the manufacture of pharmaceutical agents.

6. Use of the compounds according to formula I of at least one of claims 1-4 for the manufacture of pharmaceutical agents for treating inflammatory diseases.

7. Process for the manufacture of intermediates of general formula IV, **characterized in that** benzaldehydes of general formula II are reacted with substituted quinolone amines of formula III to imines of general formula IV in the presence of Lewis acids and/or under acidic conditions, 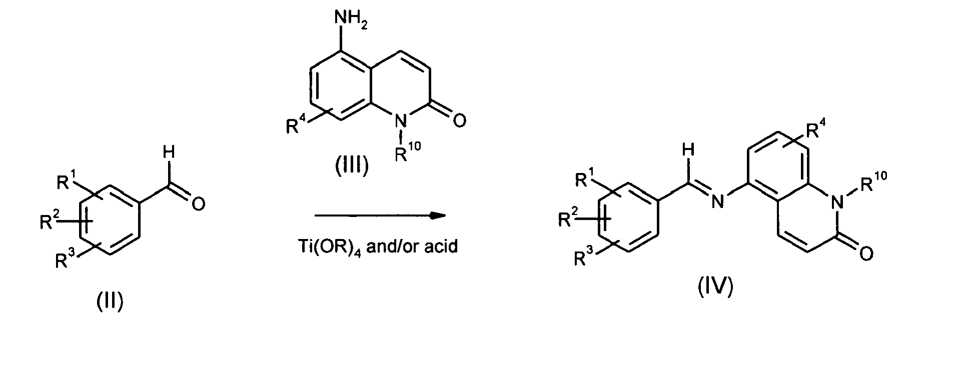 wherein R¹, R², R³, R⁴ and R¹⁰ have the meanings that are defined in claim 1 and R is C₁-C₄-alkyl.

8. Process for the manufacture of compounds of general formula I, **characterized in that** epoxides of general formula VI optionally in its enantiomerically pure form are reacted with compounds of general formula R⁵-Met
whereby Met means alkalimetals, alkaline earth metals, aluminium, copper, silicon or tin
in the presence of Lewis acids, or
are opened directly by cyanides, amines, alcohols, thioalcoholes, halogenides and/or water in the presence of bases or strong protic acids.
to yield compounds of general formula I 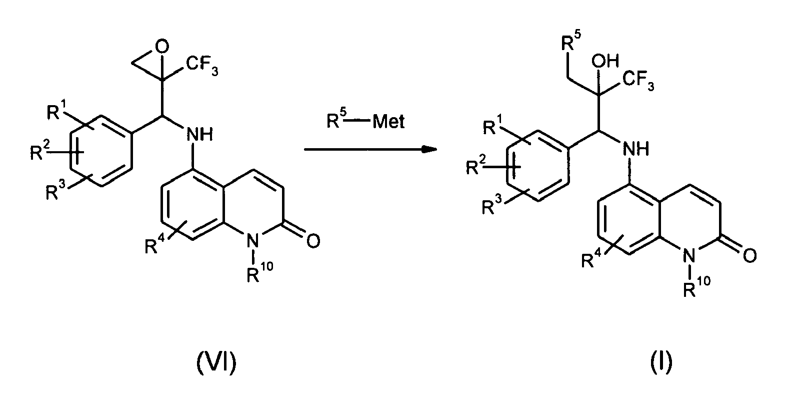 wherein R¹, R², R³, R⁴, R⁵ and R¹⁰ have the meanings that are defined in claim 1 and optionally subsequently a separation of diastereoisomers may be performed.

9. Compounds of general formula VI according to claim 8, 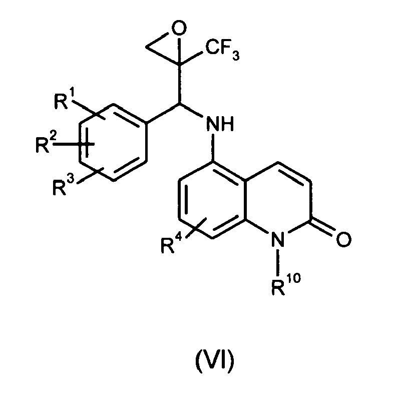 in form of a racemic mixture or as enantiomerically pure isomer.

10. Process for the manufacture of compounds of general formula I, **characterized in that** ketones of type (IX) optionally in its enantiomerically pure form can be condensed with substituted amino quinolones of type (III) to imines and subsequently or simultaneously reduced to yield compounds formula I 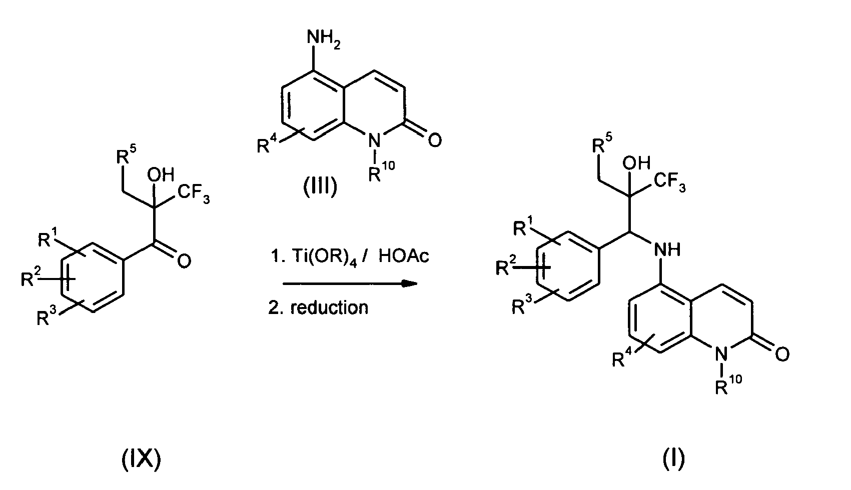 wherein R¹, R², R³, R⁴, R⁵ and R¹⁰ have the meanings that are indicated in claim 1 and R as defined in claim 10 and optionally subsequently a separation of diastereoisomers may be performed.

11. Process for the manufacture of compounds of general formula (I),
**characterized in that** compounds of type (X) optionally in its enantiomerically pure form can be reacted with arylboronic acids of type (XI) to yield compounds of formula (I)

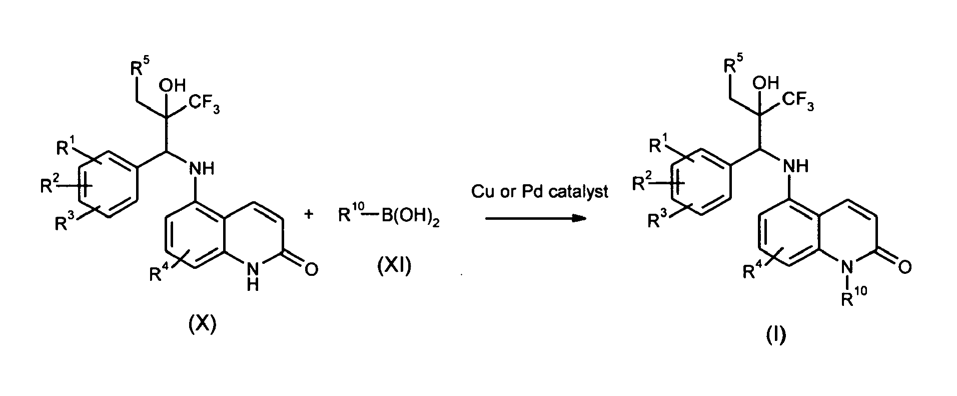

12. A pharmaceutical composition comprising a compound or formula (I) or a pharmaceutically acceptable salt thereof as defined in claim 1-4, and a pharmaceutically acceptable adjuvant, diluent or carrier.

13. A compound or formula (I), or a pharmaceutically acceptable salt thereof, as claimed in claim 1-4 for use in therapy.

14. The use of a compound or formula (I), or a pharmaceutically acceptable salt thereof, as claimed in claim 1-4, in the manufacture of a medicament for use in the treatment of a glucocorticoid receptor mediated disease state.

15. A combination of a compound of formula (I), or a pharmaceutically acceptable salt thereof, and one or more agents selected from the list comprising:
• a PDE4 inhibitor including an inhibitor of the isoform PDE4D;
• a selective β.sub2. adrenoceptor agonist such as metaproterenol, isoproterenol, isoprenaline, albuterol, salbutamol, formoterol, salmeterol, terbutaline, orciprenaline, bitolterol mesylate, pirbuterol or indacaterol;
• a muscarinic receptor antagonist (for example a M1, M2 or M3 antagonist, such as a selective M3 antagonist) such as ipratropium bromide, tiotropium bromide, oxitropium bromide, pirenzepine or telenzepine;
• a modulator of chemokine receptor function (such as a CCR1 receptor antagonist); or,
• an inhibitor of p38 kinase function.
